# EUROPEAN PATENT APPLICATION

(11) **EP 3 514 166 A1**
(43) Date of publication of application: **24.07.2019**
(21) Application number: 18382030.7
(22) Date of filing: 22.01.2018
(51) Int. Cl.: C07K 7/08, G01N 33/68, C07K 16/16

(54) **METHOD FOR THE DETECTION AND/OR QUANTIFICATION OF LUPIN BETA-CONGLUTIN ALLERGEN PROTEINS**

(71) Applicant: Consejo Superior de Investigaciones Científicas (CSIC), 28006 Madrid (ES)
(72) Inventor: Jimenez Lopez, José Carlos, 18008 Granada (ES); Alche Ramírez, Juan de Dios, 18008 Granada (ES); Lima Cabello, Elena, 18008 Granada (ES)
(74) Representative: Pons Ariño, Angel

(57) **Abstract**

The present invention provides an improved antibody, method and kit for the specific detection and/or quantification of the main allergen proteins of lupin seeds, namely beta-conglutins, preferably in foodstuffs. The method of the invention is preferably based on an ELISA assay and the antibody used has been designed against an epitope that is shared by at least seven isoforms of the beta-conglutin proteins of the *Lupinus angustifolius* species and at least one isoform of the *Lupinis albus* species. Thus, the method of the invention is highly specific and sensitive, so that it may detect and quantify even lupin traces present in food samples which might elicit allergic reactions in sensitized consumers.

## Description

The present invention falls within the fields of immunology, allergology and food industry, specifically within methods and kits suitable for the identification and/or quantification of food allergens, more particularly lupin allergen proteins, in food samples. These methods and kits are therefore useful for classifying the offending food containing hidden lupin allergens that may elicit allergic reactions in sensitized consumers.

### BACKGROUND ART

During the past decades, the prevalence of food allergies has significantly increased in industrialized countries. Since even minimal concentrations of an allergen can induce severe immunological reactions and since there is no effective therapy, the complete avoidance of the allergenic food is the only option left for allergic patients.

Lupin or lupine is a legume that belongs to the *Fabaceae* family and which is increasingly used in food industry around the world. The convenient nutritional attributes of lupin, such as high protein and dietary fibre content as well as hypoglycaemic and anticholesterolemic properties, are being recognized and technological applications are extending the use of lupin in food. As a consequence, lupin is being used in a wide range of food products in many countries, particularly in Europe. For instance, lupin bran and flour are used in staple foods, such as bread and pasta, and confectionery, as well as in dietary products such as milk for allergic people, vegetarians and celiac disease patients. In food production three (sweet) lupin species are commonly used: *Lupinus albus, Lupinus angustifolius* and *Lupinus luteus.* However, some of the lupin proteins, predominantly those in the alpha-, beta-and gamma-conglutin protein fractions, are known for being allergenic, potentially making lupin a hidden food allergen (Jappe U. and Vieths S., 2010, Mol Nutr Food Res., 54(1): 113-26).

Evidences showing allergic reactions to lupin confirm that this ingredient is a significantly important new allergen that represents a risk to allergic consumers. The public health significance of lupin as a food allergen in Europe is supported by a large body of evidence. Although severe allergic reactions (including anaphylaxis) to lupin and lupin-containing food products have been reported, the prevalence of lupin allergy in the general population is unknown.

Lupin is an emerging food allergen of public health significance and its increasing use in food would support the inclusion of this ingredient in the list of allergens subject to mandatory declaration in the product label. Indeed, lupin has been included in a list of 14 allergens (Regulation No. 78/2014) that are recognized across Europe as the most common ingredients or processing aids causing food allergies and intolerances. Any food product which contains or uses an ingredient or processing aid derived from one of the substances or products listed in these 14 allergens must be labelled and declared to the consumer. However, although there are food regulations since 2006 that recognize lupin as a significantly relevant allergen in the European Union and where it has been mandatory allergen labelling for food products containing lupin, there is a lack of information about commercial methods that allow a reliable identification and quantification of this food allergen. Since lupine is amongst these recognized allergens, analytical methods must be developed allowing the detection of traces of lupine in complex and processed foodstuffs.

Although the presence of lupin in food is currently limited, it is likely to increase and the potential for lupin as "hidden ingredient" is high. The allergenic potential of lupin and derived substances is not destroyed by common food processing methods. Furthermore, there is limited information on lupin cross-contamination in the commercial food supply. At the food manufacturing level, lupin cross-contamination needs to be managed in the same way as other food allergens to minimize inadvertent exposure by allergic consumers.

For lupine allergic persons, hidden lupine allergens in food are a critical problem. Already very low amounts of lupine can cause allergic reactions, which may lead to anaphylactic shock in severe cases. Because of this, lupine allergic persons must strictly avoid the consumption of lupine containing food. Cross-contamination, mostly as a consequence of the production process, is often noticed. This explains why in many cases the existence of lupine residues in food cannot be excluded. For this reason sensitive detection systems for lupine residues in foodstuffs are required.

DNA-based analytical methods are an important tool for the detection of certain allergens in foods, and PCR methods for the detection of lupine in food have been developed (Demmel A., et al., 2008, Journal of Agricultural and Food Chemistry, 56, 4328-4332; Gomez Galan, et al., 2010, European Food Research and Technology, 230, 597-608). In addition to PCR techniques, enzyme linked immunosorbent assays (ELISAs) are the methods of choice in allergen analysis. Three examples of sandwich ELISAs for the detection of potentially allergenic lupine proteins in foods are described in Holden, Faeste, and Egaas, 2005, Journal of Agricultural and Food Chemistry, 53, 5866-5871; Holden L., et al., 2007, Journal of Agricultural and Food Chemistry, 55, 2536-2542; and Kaw, Hefle, and Taylor, 2008, Journal of Food Science, 73, 135-140. However, all these three ELISAs suffer from some cross-reactivity, either with chick-pea, peanut, pea, soy, lentil, fenugreek, almond, cashew, sunflower seed, pumpkin seed or black bean. In addition, all these three methods include time consuming sample extraction procedures.

The development and validation of another sandwich ELISA for the determination of traces of lupine allergenic proteins in food has been disclosed in Christina Ecker and Margit Cichna-Mark, 2012, Food Chemistry, 130: 759-766. This ELISA method detected proteins from white (*Lupinus albus*) and blue (*Lupinus angustifolius*) lupine and, with a lower sensitivity, proteins from yellow (*Lupinus luteus*) lupine. Nevertheless, a disadvantage of this method is that the anti-lupin antibodies used were produced by immunizing a rabbit and a hen with a whole protein extract from white lupine flour. This means that the antibody is not only specific for allergenic lupin proteins but it may also recognize other non-allergenic proteins also present in the whole extract used for the immunization, thus providing false positives when used to test the presence of lupin allergenic proteins in food samples.

Additionally, colorimetric enzyme immunoassays for the quantitative determination of lupin in food (*i*. *e*. Lupin ELISA kits) are commercially available, examples of which are Product Nos. ABIN2683196, ABIN997094, DELUPE01 from Demeditec Diagnostics, or Lupine BioAssay™ ELISA Kit (Product No. L6000-01-1 Kit) from United States Biological. Most of these kits are capable of quantifying lupin residues in food products such as sausage, bakery products, potato products, ketchup and juices. However, the same problem as that indicated above applies to these commercial kits, *i*. *e*. they use antibodies that were generated against whole protein extracts from lupin seeds, which means that they may be specific for non-allergenic proteins also present, together with the allergenic proteins, within the lupin extract used for immunization. Consequently, these commercial kits detect in fact a broad set of lupin proteins within the food sample some of which may be allergenic whereas others are not. The reliability of these kits is therefore low.

Since increasing the food safety is a current issue, and the protection of consumers with lupin food allergy from the danger of this hidden allergen is an important part of this goal, improved methods and kits for the reliable and sensitive detection and/or quantification of lupin allergenic proteins in foodstuffs are still needed.

### DESCRIPTION OF THE INVENTION

The present invention provides an improved antibody, method and kit for the specific detection and/or quantification of the main allergen proteins of lupin seeds, namely beta-conglutins (or Lup an 1 allergen or conglutin β1 proteins), preferably in foodstuffs.

The main advantage of the present invention is that the designed antibody used in the method described herein is specific for an immunogenic peptide (epitope of SEQ ID NO: 1) shared by seven isoforms of the main lupin allergen proteins, beta-conglutins, of *L. angustifolius* and also by one of the isoforms of the *L. albus* beta-conglutins. This shared epitope within allergenic beta-conglutin isoforms had not been identified nor disclosed before. Thus, this antibody specifically recognizes the main allergenic proteins of at least these two lupin species commonly used in food industry. The method described in the present invention is therefore more specific, reliable and sensitive than those disclosed in the art wherein antibodies against a whole lupin protein extract, which may contain allergenic and non-allergenic proteins, are used.

Furthermore, the method presented herein is sensitive enough so as to detect even lupin protein traces (cross-contamination) in foods which might elicit allergic reactions in sensitized consumers.

The method disclosed in this invention will help classifying and labeling the presence of lupin allergens in food. Providing this information for prepacked food and non-prepacked food is currently mandatory according to the European Commission Delegated Regulation No. 78/2014. Moreover, the method is suitable for applications in the food industry such as the control of raw ingredients, processing equipment, and final products for contamination, as well as for food surveys commissioned by food authorities.

Regarding the epitope of SEQ ID NO: 1 disclosed in the present invention, which was used by the inventors to generate the antibody applied to the method disclosed herein, it has been identified in the present invention based on the beta-conglutin sequences of *L. angustifolius* and *L. albus* and based on three biochemical properties which are of great importance for the immunogenic and allergenic activities of a peptide. These properties are hydrophobicity, polarity and volume of the lateral amino acid chains.

Thus, a first aspect of the present invention refers to an isolated amino acid sequence consisting of SEQ ID NO: 1. This amino acid sequence of SEQ ID NO: 1 is the "epitope of the invention", which is shared by at least seven isoforms of the beta-conglutin protein of *L. angustifolius* and at least one of the isoforms of the beta-conglutin protein of *L. albus.* SEQ ID NO: 1 is the amino acid sequence VDEGEGNYELVGIR.

The term "beta-conglutin" or "conglutin β1" refers to the lupin protein family also named "Lup an 1 allergen". It is the major lupin allergen. Beta-conglutins are storage proteins and the most abundant protein fraction in lupin seeds. It is the most variable (polymorphic) family in terms of gene and protein sequence, which may be reflected in differential functionalities and in allergenicity. Preferred beta-conglutins referred to in the present invention include the seven beta-conglutin isoforms from *L. angustifolius* which are: b1 (Uniprot accession number: F5B8V9, SEQ ID NO: 2), b2 (Uniprot accession number: F5B8W0, SEQ ID NO: 3), b3 (Uniprot accession number: F5B8W1, SEQ ID NO: 4), b4 (Uniprot accession number: F5B8W2, SEQ ID NO: 5), b5 (Uniprot accession number: F5B8W3, SEQ ID NO: 6), b6 (Uniprot accession number: F5B8W4, SEQ ID NO: 7), and b7 (Uniprot accession number: F5B8W5, SEQ ID NO: 8); and one beta-conglutin isoform from *L. albus* which is: b1 (Uniprot accession number: Q6EBC1, SEQ ID NO: 9).

The term "epitope" refers to the antigen individual portion capable of eliciting an immunologic reaction. The epitope is the antigen portion that is specifically recognized by the antibody.

The epitope of the invention may be synthesized, for example, but not limited to, *in vitro.* For example, through the synthesis of peptides in solid phase or by means of recombinant DNA approaches. The epitope of the invention can be recombinantly produced, directly or as a fusion polypeptide together with a heterologous polypeptide which may contain, for example, but not limited to, a signal sequence or other polypeptide having a protease cleavage site, for example, but not limited to, at the N-terminal end of the mature peptide. The recombinant production of the epitope of the invention may comprise the amplification of a polynucleotide that encodes for said epitope, the cloning of the amplified polynucleotide in an expression vector, the transformation or transfection of a competent cell with said vector, the culture of said cell under conditions that promote the expression in the epitope of the invention and the isolation and purification of the epitope of the invention produced by the cell.

Thus, another aspect of the invention refers to an isolated nucleic acid sequence encoding the epitope of the invention, hereinafter the "nucleic acid sequence of the invention". Another aspect of the invention refers to a gene construction, hereinafter the "gene construction of the invention", comprising this nucleic acid sequence of the invention. In a preferred embodiment, the gene construction of the invention is an expression vector. Another aspect of the invention refers to an isolated host cell comprising the nucleic acid sequence or the gene construction of the invention, hereinafter the "host cell of the invention".

In accordance with the present invention, "an isolated nucleic acid molecule", "nucleotide sequence", "nucleic acid sequence" or "polynucleotide" is a single or double stranded nucleic acid molecule (polynucleotide) that has been preferably removed from its natural milieu (i.e., that has been subject to human manipulation) and can include DNA, RNA, or derivatives of either DNA or RNA, including cDNA. The nucleotide sequence of the present invention can be or not chemically or biochemically modified and can be artificially designed by means of cloning and selection methods or by sequencing. The nucleic acid sequence of the invention can encode the mature epitope of the invention or a peptide consisting of a signal peptide linked to the epitope of the invention which will have to be subsequently processed.

The nucleotide sequence of the present invention can also comprise other elements, such as intron/s, promoter/s, enhancer/s and/or terminator sequence/s, stop codon/s for transcription and/or translation, non-coding sequences at 3'and/or 5'ends, ribosome binding site/s, etc. This nucleotide sequence can also include coding sequences for additional amino acids which are useful for the stability or purification of the encoded peptide.

The gene construction of the invention may further comprise one or more gene expression regulatory sequences or control sequences, such as leaders, promoters, terminators, polyadenylation sequences, replication origin/s, etc.

The term "expression vector" refers to a DNA molecule, linear or circular, that comprises the nucleic acid sequence of the invention operably linked to additional segments provided for the successful transcription of the encoded peptide. Generally a plasmid is used as expression vector to introduce a specific nucleic acid sequence into a target cell. Once the expression vector is inside the cell, the peptide that is encoded by the nucleic acid sequence is produced by the cellular-transcription and translation machinery of the ribosomal complexes. The plasmid is frequently engineered to comprise regulatory sequences that act as enhancer and promoter regions and lead to an efficient transcription of the nucleic acid sequence carried on the expression vector. The expression vector of the invention is introduced into a host cell so that the vector is maintained as a chromosomal integrant or as a self-replicating extra-chromosomal vector. Examples of expression vectors are, but without limitations, plasmids, phages, cosmids, phagemids, yeast artificial chromosomes (YAC), bacterial artificial chromosomes (BAC), human artificial chromosomes (HAC), or viral vectors, such as adenovirus, retrovirus or lentivirus.

The epitope of the invention has antigenic capacity. Thus, it may be used to immunize an individual, preferably a mammal, more preferably a rabbit, in order to obtain antibodies that specifically bind it. Immunization may be performed, for instance, injecting to the individual the epitope of the invention together with an adjuvant. Adjuvants useful for this purpose are any of those known in the art for vaccination, such as complete or incomplete Freund's adjuvant. One or more doses of the epitope may be administered to the individual over the time. One or more memory doses may be also administered. Once the immunization takes place, serum from the immunized individual is collected. The extraction of the serum may be performed, for example, but not limited to, by means of blood extraction, subsequent coagulation thereof, removal of the fibrin clot and other components, and isolation of the serum. In order to obtain the serum, anticoagulant is not applied to the blood, but rather it is left to coagulate and is centrifuged. "Serum" or "blood serum" is understood to be the blood component resulting after allowing the blood coagulation and removing the fibrin clot and other components which contain a number of biological effectors such as the platelet-derived growth factor, secreted by the cellular components during said coagulation. It is yellow in colour, brighter than plasma. Subsequently, the antibodies present in the serum, which are specific against the epitope of the invention used for the immunization, are purified. Various antibody purification methods that could be carried out for such purpose are known in the state of the art, such as for example, but not limited to, electrophoretic or chromatographic affinity methods.

The epitope of the invention can also be used to immunize a bird. Preferably, after being immunized, the eggs of the immunized bird are collected, the yolks are separated, the lipids removed and the antibodies precipitated/purified using one of the various methods known in art.

"Electrophoresis" is an analytical separation technique based on the movement or migration of macromolecules dissolved in a medium (electrophoresis buffer), by means of a matrix or solid support as a result of the action of an electrical field. The behavior of the molecule depends on its electrophoretic mobility and this mobility depends on charge, size and shape. There are numerous variations of this technique based on the equipment used, supports and conditions for carrying out the separation of the molecules. Electrophoresis techniques are, preferably, selected from the list consisting of: capillary electrophoresis, paper electrophoresis, agarose or polyacrylamide gel electrophoresis, isoelectric focusing or bidimensional electrophoresis.

"Chromatographic methods" allow the separation of the molecules according, but not limited to, their charge, size, molecular mass, polarity or redox potential. Chromatographic techniques are, preferably, selected from: partition chromatography, filtration, adsorption chromatography, molecular exclusion chromatography, ionic exchange chromatography, hydrophobic chromatography or affinity chromatography, and can be executed on columns, paper or plates.

Another aspect of the invention refers to the *in vitro* use of the epitope of the invention for the production and/or design of antibodies or aptamers against beta-conglutin allergen proteins of lupin. This *in vitro* use of the epitope of the invention may be performed by, for instance, predictive bioinformatics tools. Another aspect of the invention refers to the use of the epitope of the invention as an immunogen for the immunization of an individual to produce an antibody against beta-conglutin allergen proteins of lupin.

The term "lupin", "Lupinus" or "lupine" refers to the genus of flowering plants in the legume family *Fabaceae.* This term, as used in the present invention, includes the species *L. albus, L. angustifolius* and *L. luteus,* preferably *L. albus* and *L. angustifolius,* more preferably *L. angustifolius.* White lupine (*Lupinus albus*) shows a high content of essential amino acids and is particularly cultivated for food production, whereas blue (*Lupinus angustifolius*) and yellow (*Lupinus luteus*) lupine are predominantly used for feed. The role of *L. angustifolius* in the food industry is, however, increasing. "Sweet lupins" include these *"Lupinus angustifolius"* or "Narrow-leafed lupin (NLL)" or "blue lupin"; "*L*. *albus*" or "white lupin"; and "*L*. *luteus*" or "yellow lupin".

The term "individual", as used herein, refers to an animal, preferably a bird or a mammal, more preferably a mammal, even more preferably a non-human mammal. Examples of individuals that could be immunized with the epitope of the invention in order to produce an antibody against lupin beta-conglutin allergen proteins are mouse, rabbit, rat, horse, monkey, donkey, dog, cat, pig, guinea pig, goat, sheep, chicken, hen, hamster, and the like. Preferably, in the present invention the individual to be immunized is a mouse or a rabbit, more preferably a rabbit.

Another aspect of the invention refers to a method for producing antibodies against lupin beta-conglutin allergen proteins which comprises:
i. Collecting the serum of an individual previously immunized with the epitope of the invention, and
ii. Purifying the antibodies against the epitope of the invention present in the serum collected in step (i).

Another aspect of the invention refers to the serum or antiserum obtained by the method explained in the paragraph above. This antiserum comprises antibodies against the epitope of the invention, i. e. against lupin beta-conglutin allergen proteins.

Another aspect of the invention refers to an aptamer that specifically binds to the epitope of the invention. The term "aptamer" refers to a single stranded oligonucleotide (ssDNA or RNA) or peptide that specifically binds to a target molecule. Aptamers are usually created by selecting them from a large random sequence pool (sequence library) by the SELEX method in the presence of the ligand. The SELEX method allows selecting from the library the oligonucleotide or peptide that binds to the target molecule with the highest affinity. Aptamers can be combined with ribozymes to self-cleave in the presence of their target molecule. Adding an unnatural base to a standard aptamer can increase its ability to bind target molecules. Oligonucleotide aptamers have a central region with a random sequence and two flanking regions of known sequence. The total length of the aptamer may be, for instance, between 70 and 100 bp. The length of the central region may be, for instance, between 30 and 60 bp.

Another aspect of the invention refers to an antibody, hereinafter the "antibody of the invention", that specifically binds the epitope of the invention. Preferably, this antibody of the invention has been obtained by the method for producing antibodies against lupin beta-conglutin allergen proteins explained above. In another preferred embodiment, the antibody of the invention is a rabbit antibody, which means that it has been obtained (purified) from the serum of a rabbit previously immunized with the epitope of the invention.

The expressions antibody "against the", "that reacts to", "specific for", "that specifically binds" or "that recognizes" the epitope or the invention, can be used interchangeably.

In another preferred embodiment, the minimum amount of epitope of the invention that can be detected by the antibody of the invention is 0.07 ng of epitope of the invention. This affinity of the antibody of the invention for the epitope of the invention can be measured, for instance but without limitation, with an ELISA assay.

The term "antibody" relates to immunoglobulin molecules or to immunologically active portions of immunoglobulin molecules, i.e. molecules containing an antigen fixation site which specifically binds (reacts) to the epitope of the invention. Examples of immunologically active portions of immunoglobulin molecules include fragments F(ab) and F(ab')2, which may be generated by treating the antibody with an enzyme such as pepsin or recombinantly. The antibodies mentioned in the present invention are, preferably, IgG.

The antibody of the invention may be a monoclonal or a polyclonal antibody, and it is specific for a single determinant -epitope- in the antigen. Preferably, it is a polyclonal antibody, as it is produced using a synthetic peptide corresponding to the single epitope of the invention with the SEQ ID NO: 1 as an immunogen for the immunization of an individual. This antibody contains only one species of an antigenic fixation site capable of immune reacting with this particular epitope (SEQ ID NO: 1) within the antigen. This antibody may be biochemically altered by means of genetic manipulation or may be synthetic, possibly lacking, in its entirety or in parts, portions that are not necessary for recognizing the epitope of the invention, and being substituted by others that transmit additional advantageous properties to the antibody.

The antibody of the invention may also be recombinant, humanized, chimeric, synthetic or any combination thereof.

A "recombinant antibody" (rAC) is an antibody which has been produced in a host cell which has been transformed or transfected with the nucleic acid sequence of the invention that encodes the epitope of the invention, with a nucleic acid sequence encoding the antibody of the invention, or that produces the epitope of the invention or the antibody of the invention as a result of an homologous recombination. Said host cell includes a cell in an *in vitro* cell culture and a cell in a host animal.

The antibody of the invention may also be "chimeric". Thus, a region of the heavy and/or light chain is identical or homologous to the corresponding sequences in antibodies from a certain species or belonging to a class or subclass of certain antibodies, while the remaining chain(s) are identical or homologous to the corresponding sequences in antibodies derived from another species or belonging to another class or subclass of antibodies, or to fragments of said antibodies, in such a manner as to exhibit the desired biological activity.

The antibody of the invention may be labeled (i. e. conjugated with a detection system) and/or immobilized in a support. Preferably, the antibody of the invention is conjugated with a detection system. More preferably, the detection system is a dye, fluorochrome or enzyme, even more preferably an enzyme.

The term "labeled" or "conjugated with a detection system" means that the antibody is conjugated with (linked to) a label. A "label" is any compound or molecule capable of giving rise to a chromogenic, fluorogenic, magnetic, electrodense, radioactive and/or chemiluminescent signal, allowing the detection of the labeled antibody. Labels that may be conjugated to an antibody are well known in the state of the art, for instance but without limitations, a radioisotope [for example, 32P, 35S or 3H], a fluorescence or luminescent marker (fluorochrome) [for example, GFP, m-cherry, fluorescein (FITC), rhodamine, texas red, phycoerythrin (PE), alophycocyanin, 6-carboxyfluorescein (6-FAM), 2',7'-dimethyl-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), and the like], magnetic particles or electrodense particles (gold, ferritin, etc.), a secondary antibody (labeled or not) or fragments thereof [for example, F(ab)2) fragments], an affinity label [for example, biotin, avidin, agarose, bone morphogenetic protein (BMP), haptens], an enzyme or a substrate of an enzyme [for example, alkaline phosphatase (AP) or horseradish peroxidase (HRP)], a dye [for example, dylight488 and the like], or Amine-modified linker (S-HyNic, succinimidyl-6-hydrazino-nicotinamide linker; S-4FB, succinimidyl-4-formylbenzamide linker). In a particular embodiment, the label or detection system to which the antibody of the invention is conjugated is the enzyme HRP.

The label can be bound to the antibody directly or it may be indirectly bound by means of another compound.

Furthermore, the antibody of the invention may be linked to an affinity tag for its purification, preferably to a polyhistidine-tag. Thus, in another preferred embodiment, the antibody of the invention is linked to a 6xHis-Tag in its N- or C-terminal end, more preferably in its C-terminal end.

The antibody of the invention may also be immobilized on a support, for instance, a matrix surface (preferably a nylon matrix), a membrane, a glass or plastic support, for example a microtiter plate, nanotechnology-based supports, carbon derivatives and others, or on beads for example agarose spheres or superparamagnetic microspheres formed by biodegradable matrixes. The term "immobilized" means that the antibody is linked to a support without losing its activity.

Another aspect of the invention refers to a kit, hereinafter the "kit of the invention", comprising the antibody of the invention and the following polypeptides:
a. the beta-conglutin isoforms comprising SEQ ID NO: 2, SEQ ID NO: 3, SEQ ID NO: 4, SEQ ID NO: 5, SEQ ID NO: 6, SEQ ID NO: 7 and SEQ ID NO: 8, and/or
b. the beta-conglutin isoform comprising SEQ ID NO: 9.

In a preferred embodiment, the kit of the invention comprises all the beta-conglutin isoforms indicated in (a) and (b) above.

In another preferred embodiment, the kit of the invention comprises a support. Supports comprised in this kit may be any of those indicated above, more preferably the support is a plastic support, even more preferably a microtiter plate. In a particular embodiment, the support is a 96 wells microtiter plate.

Isoforms indicated in (a) and (b) above comprised in the kit are also called in this invention "quantification standards", since they are used to generate the standard quantification curve as it will be explained below.

These quantification standards can be obtained by natural isolation from lupin seeds or they may be obtained by DNA recombinant technology, preferably using bacteria as host cells. Methods for obtaining recombinant proteins are well known by the skilled in the art.

SEQ ID NOs: 2, 3, 4, 5, 6, 7 and 8 correspond to beta-conglutins from *L*. *angustifolius,* and SEQ ID NO: 9 corresponds to the beta-conglutin from *L*. *albus,* as explained previously in this description.

These quantification standards may be labeled and/or immobilized in the kit of the invention, preferably labeled. Labels and supports that may be used for this purpose are the same as those indicated previously in this description. Each one of the isoforms or quantification standards indicated in (a) and (b) above is preferably present in the kit of the invention in form of different known serial dilutions. The term "dilutions" refers to stepwise serial dilutions of the isoforms in solution, wherein the dilution factor at each step and for the different isoforms is constant, resulting in a geometric progression of the concentration of the isoforms in a logarithmic fashion. An example of dilution factor that could be applied would be a ten-fold serial dilution which could be 1 M, 0.1 M, 0.01 M, or 1 mM, 0.1 mM, 0.01 mM, 0.001 mM, etc. Serial dilutions are used to accurately create highly diluted solutions as well as solutions for assays resulting in concentration curves with a logarithmic scale. In the present invention, each one of the serial dilutions of the quantification standards is quantified, preferably each time the method of the invention is performed, in order to generate a standard curve against which signals detected in the problem samples are compared. This helps to determine the amount of allergen present in each one of the problem samples. Samples comprising serial dilutions of a known allergen are widely used for the preparation of standard curves in processes for the quantification of allergens in samples, thus a skilled in the art will know how to carry them out.

Preferably, each one of the isoforms is present in the kit of the invention in an amount between 0.07 ng and 1,000 ng, more preferably in an amount of 12.5 ng.

Preferably, each one of the diluted isoforms is present in the kit of the invention in an amount between 0.01 µL and 1,000 µL, more preferably in an amount of 100 µL.

The antibody of the invention or the quantification standards may be free or immobilized, or both, in the kit of the invention. More preferably, the antibody of the invention or the quantification standards are immobilized in the support comprised in the kit of the invention. For instance, the antibody of the invention is immobilized in each of the wells of the microtiter plate comprised in the kit or the quantification standards are immobilized in some of the wells of the microtiter plate comprised in the kit.

In another preferred embodiment, the kit of the invention further comprises a secondary antibody, which preferably binds to the antibody of the invention and more preferably is conjugated with a detection system. Even more preferably, this secondary antibody is an anti-rabbit antibody. Detection systems to which this secondary antibody may be conjugated are the same as those previously indicated in this description. Preferably, this secondary antibody comprised in the kit of the invention is conjugated with the enzyme HRP.

In addition, the kit of the invention may optionally comprise a tertiary antibody that could be used in case the secondary antibody comprised in the kit is not conjugated. Preferably, this tertiary antibody is an anti-rabbit antibody. More preferably, this tertiary antibody binds to the secondary antibody comprised in the kit and more preferably is conjugated. Detection systems to which this tertiary antibody may be conjugated are the same as those previously indicated in this description. Preferably, this tertiary antibody comprised in the kit of the invention is conjugated with the enzyme HRP.

In a more preferred embodiment, the kit of the invention further comprises reactive sticks, flurochrome/s, dye/s, substrate/s specific for the detection system selected to which the antibodies comprised in the kit are conjugated (this substrate/s are required for initiating the labeling reaction), blocking buffers, extraction buffers, washing solutions and/or stop solutions.

"Substrate/s required for initiating the labeling reaction" are those compatible with the detection or conjugation system chosen. These substrate are those whose binding to their target (for instance, enzyme) triggers the reaction (for instance, enzymatic reaction) by which a detectable signal (for instance, chemiluminescence, fluorescence or colour) is produced. In another preferred embodiment, the substrate required for initiating the labeling reaction comprised in the kit of the invention is 5-bromo-4-chloro-3-indolyl-phosphate/Nitro Blue Tetrazolium (NBT/BCIP) or Tetramethylbenzidine (TMB), more preferably TMB. TMB is the substrate of the HRP enzyme. TMB is a chromogenic substrate used in staining procedures in immunohistochemistry as well as being a visualizing reagent used in enzyme-linked immunosorbent assays (ELISA). TMB is a white crystal powder that forms a pale blue-green liquid in solution with ethyl acetate. TMB acts as a hydrogen donor for the reduction of hydrogen peroxide to water by peroxidase enzymes such as HRP.

The "blocking buffer" or "blocking solution" optionally comprised in the kit of the invention is any buffer capable of blocking the protein sites not bound to an antibody with which they have been previously incubated. The preferred blocking buffer used in the present invention comprises non-fat dry milk, more preferably together with PBS, even more preferably 5% non-fat dry milk/PBS. Other less preferred blocking buffers that could also be used alternatively are, for example, StartingBlock, SuperBlock, BSA, Casein, BLOTTO (non-fat dry milk proteins in TBS), Pierce Clear Milk, Pierce Fast, Sea Block or Protein-Free.

The "washing solution" optionally comprised in the kit of the invention is any buffer capable of removing the unbound material to a support. The preferred washing solution used in the present invention is PBS, although other washing solutions such as Tween-20, TRIZMA BASE, Tris-HCI, TBS or Triton-X could also be used as alternatives or in combination with the preferred one.

The "stop solution" optionally comprised in the kit of the invention is any buffer capable of stopping the labeling reaction. The preferred stop solution used in the present invention is 0.6 N sulphuric acid, more preferably at 1M, or alternatively equal volumes of 1 N HCl and 0.6 N sulfuric acid. In the presence of HRP enzyme conjugates, TMB and peroxide react to produce a blue byproduct having maximum absorbance at 605nm. The color intensity is proportional to the amount of HRP activity, which in turn is related to the levels of target analyte in an optimized ELISA procedure. Addition of sulfuric acid stop solution changes the color to yellow (absorbance maximum at 450nm), stabilizing the color development to enable accurate measurement of the intensity at 450nm using a spectrophotometer or plate reader.

The "extraction buffer" optionally comprised in the kit of the invention is any buffer capable of extracting proteins, preferably allergen proteins, more preferably beta-conglutin proteins, from lupin-containing samples. The preferred extraction buffer used in the present invention comprises Tris (Tris(hydroxymethyl)aminomethane, (HOCH₂)3CNH₂) preferably 100 mM and pH 7.4; NaCl preferably 150 mM; EGTA preferably 1 mM; EDTA preferably 1 mM; Triton X-100 preferably 1%; Sodium deoxycholate preferably 0.5%; a protease inhibitor cocktail and PMSF (phenylmethylsulfonyl fluoride) preferably 1 mM.

The kit of the invention may also comprise all the elements needed for carrying out the method of the invention disclosed below. These additional elements may include, but without limitations, agents to prevent the contamination and/or proteins, peptides or antibodies that act as positive or negative controls. The kit of the invention may also comprise the instructions to carry out the method of the invention explained below.

Another aspect of the invention refers to the use of the antibody of the invention or the kit of the invention for the *in vitro* detection and/or quantification of lupin allergen proteins, preferably lupin beta-conglutin allergen proteins, more preferably *L. angustifolius* and/or *L. albus* beta-conglutin allergen proteins, even more preferably beta-conglutin allergen proteins comprising SEQ ID NOs: 2, 3, 4, 5, 6, 7, 8 and/or 9. In another preferred embodiment, this *in vitro* detection and/or quantification is performed in food samples or in biological samples, more preferably in food samples.

"Biological samples" are those samples that have been isolated from or derive from any part of the lupin plant, such as leaves, root, seed or stem, preferably seed.

"Food samples" are those samples that have been isolated from foodstuff. This term includes non-processed (raw ingredients) and processed food products. "Processed food products" are those which have been subjected to a food processing method, including the transformation of cooked ingredients by physical or chemical means into food, or of food into other forms. Food processing combines raw food ingredients to produce marketable food products that can be easily prepared and served by the consumer. Food processing typically involves activities such as mincing and macerating, liquefaction, emulsification, and cooking (such as boiling, broiling, frying, or grilling); pickling, pasteurization, and many other kinds of preservation; and canning or other packaging. Primary-processing methods such as dicing, slicing, freezing or drying when leading to secondary products are also included within this definition. The term "food samples" also includes samples taken from processing equipment used during food production and/or treatment.

Food or biological samples referred to in the present invention may be in any state or presentation form. Thus, they may be fresh or frozen. They may be, but without limitation, in liquid, solid, cream, semisolid (gel), aerosol, emulsion, solution or granular form. Food samples may be, but without limitation, in form of flour, pasta, bread, bran, cake, ice-cream, dessert, or the like. Food samples may have been taken from, for instance but without limitation, pasta, confectionery, bread, biscuits or any other bakery product, flour, butter, chocolate and cocoa derivatives, sauces, potatoes, cheese, snacks, meats, beans (for instance, lupin beans), drinks (for instance, juice or milk), yogurts, salads, vegans products, oils, soups, seeds and/or grains based mix and bar, tortillas-based legumes, spreads products, fermented-based products, lupins coffee, syrups, or others.

This *in vitro* detection and/or quantification is preferably carried out by immunoassay, more preferably by ELISA.

An "immunoassay" or "immunochemical assay" is a biochemical assay that detects and/or quantifies the amount or quantity (preferably concentration, more preferably in ppm or ng/µL of sample) of one or more peptides (in the context of the present invention, lupin allergens, preferably lupin beta conglutin allergen proteins) of interest in a sample. For this purpose, the reaction uses one or more antibodies specific for the antigen/s to be detected. The quantification of the protein to be detected may be performed by any of the methods known in the art, such as the labeling of the antibody/ies or the antigen/s. The immunoassay may be competitive or non-competitive. In a competitive immunoassay the signal detected will be inversely proportional to the concentration of antigen in the sample. In a non-competitive assay (such as an "ELISA sandwich assay") the signal detected is directly proportional to the concentration of antigen in the sample. Examples of immunoassays are, but not limited to: immunoblotting (Western blot), enzyme-linked immunosorbent assay (ELISA), line immunoassay (LIA), radioimmunoassay (RIA), immunofluorescence, immunogold labeling (transmission electron microscopy), x-map or protein or lipopolysaccharide chips (LPS), real-time immunoquantitative PCR (iqPCR), electrochemiluminescent tags, label-free immunoassays (i.e. surface plasmon resonance, etc.), photoacoustic immunoassay, cloned enzyme donor immunoassay (CEDIA), lateral flow immunochromatographic assays, magnetic immunoassay (MIA), surround optical-fiber immunoassay (SOFIA), compact disk/digital versatile disk (CD/DVD) based immunoassay, or agglutination-PCR (ADAP).

In the present invention the immunoassay is, preferably, Western blot, ELISA or immunofluorescence, more preferably ELISA. ELISA methods that could be carried out in the present invention are direct ELISA, indirect ELISA or sandwich ELISA.

ELISA is based on the assumption that an immunoreagent (antigen or antibody) can be immobilized on a solid support, then putting this system in contact with a fluid phase containing the complementary reagent that can be bound to a marker compound (label).

As a way of example, a typical ELISA method that could be followed in the present invention comprises:
(a) coating a solid support with the sample to be tested;
(b) incubating the coated support of step (a) with the antibody of the invention under conditions allowing the formation of an immunocomplex with the antibody, wherein said antibody of the invention may or not be conjugated with a detection system;
(c) adding to the mixture of the previous step the substrate that initiates the labeling reaction, or
(c') in case the antibody of the invention is not conjugated in the previous step (b), the reaction is incubated with a secondary antibody which is conjugated with a detection system, wherein this secondary antibody is specific against the antibody of the invention, and the substrate that initiates the labeling reaction is added; and
(d) detecting and/or quantifying the signal obtained as a result of the labeling reaction in the previous step (c) or (c').

However, variations of this method are also comprised within this invention. An example of such a variation could be:
(a) coating a solid support with the antibody of the invention;
(b) incubating the coated support of step (a) with the sample to be tested under conditions allowing the formation of an immunocomplex with the antibody;
(c) adding again to the mixture of the previous step the antibody of the invention and incubating this mixture, wherein the antibody of the invention may be conjugated with a detection system or not;
(d) in case the antibody of the invention is conjugated in the previous step, the substrate that initiates the labeling reaction is added to the mixture,
(d') in case the antibody of the invention is not conjugated in the previous step (c), a secondary antibody is added which is conjugated with a detection system and wherein this secondary antibody is specific for the antibody of the invention, afterwards the substrate that initiates the labeling reaction is added to the mixture; and
(e) detecting and/or quantifying the signal obtained as a result of the labeling reaction in the previous step (d) or (d').

The signal produced as a consequence of the labeling reaction with the antibody conjugated with a detection system may be measured in the present invention, for instance but without limitation, by spectrophotometry, preferably at 450 nm, chemiluminiscence detection and quantification, spectrofluorometric detection and quantification, bioluminescence, differential calorimetry, analytical ultracentrifugation, interferometry, etc.

Another aspect of the invention refers to an *in vitro* method, hereinafter the "method of the invention", for the detection and/or quantification of the lupin allergen proteins, preferably lupin beta-conglutin allergen proteins, more preferably *L. angustifolius* and/or *L. albus* beta-conglutin allergen proteins, even more preferably beta-conglutin allergen proteins comprising SEQ ID NOs: 2, 3, 4, 5, 6, 7, 8, and/or 9, in isolated samples, preferably in food samples, wherein said method comprises the following steps:
a. putting in contact the isolated sample with the antibody of the invention or the kit of the invention (preferably, with the antibody of the invention comprised in the kit of the invention),
b. incubating the mixture of step (a) under conditions that allow the binding of the antibody to its antigen, and
c. detecting and/or quantifying the binding of step (b).

Variations of this method, in the sense previously explained in the present invention, are also comprised within the scope of the invention.

The expressions "putting in contact" and "incubating the mixture under conditions that allow the binding of the antibody to its antigen (epitope)", mean that the sample and the antibody of the invention, wherein said antibody is preferably comprised in the kit of the invention, more preferably it is immobilized on the support comprised in the kit of the invention as explained above, are contacted and incubated under conditions that allow the formation of antigen-antibody complexes. The mixture is incubated under these conditions during a period of time that is adequate for the immune reaction to be triggered. Those skilled in the art will know which conditions and period of time are suitable for this purpose. Preferably, incubation steps with the sample and the antibody of the invention referred to in the present invention take place during a period of time comprised between 20 min and 12 hours. In another preferred embodiment, these incubation steps take place at a room temperature (RT) or at a temperature comprised between 25 and 4°C, more preferably at 4 °C. In an even more preferred embodiment of the method of the invention, these incubation steps take place during 20 min at RT or during 12 hours at 4°C, even more preferably during 12 hours at 4 °C.

In another preferred embodiment, the detection and/or quantification steps referred to in the present invention are performed by immunoassay. In a more preferred embodiment, the immunoassay is selected from the list consisting of: Western blot, ELISA or immunofluorescence, even more preferably from ELISA or Western blot. In a particular embodiment, the immunoassay is ELISA.

Depending on the type of ELISA selected for performing the method of the invention (direct ELISA, indirect ELISA or sandwich ELISA) a secondary and/or a tertiary antibody should be used, in addition to the antibody of the invention. These antibodies are those previously disclosed in the present invention.

In another preferred embodiment, the method of the invention further comprises the following steps:
d. putting in contact different known dilutions (serial dilutions) of each one of the isoforms comprised in the kit of the invention with the antibody of the invention,
e. incubating the mixture of step (d) under conditions that allow the binding of the antibody to its antigen,
f. detecting and/or quantifying the binding of step (e),
g. generating an standard curve with the detection and/or quantification values obtained in step (f), and
h. comparing the detection and/or quantification values (preferably the absorbance values) obtained in step (c) with the standard curve generated in step (g).

Preferably, steps (d) to (f) above are run at the same time as the steps (a) to (c) in the method of the invention. That means that, preferably, problem samples and standard samples are run at the same time in the method of the invention. More preferably, the problem samples and the standard samples are run in duplicate or triplicate.

"Problem samples" are those with an unknown presence or amount of lupin beta-conglutin allergen proteins, i.e. those samples to be tested, for which detection and/or quantification of these allergen proteins need to be determined.

"Standard samples" are those comprising the beta-conglutin isoforms comprised in the kit of the invention and also called "quantification standards", preferably diluted (serial dilutions).

A "standard curve", also known as a "calibration curve", is a type of graph used as a quantitative research technique. Multiple samples with known properties ("standard samples"), in the context of the present invention with known amounts of lupin beta-conglutin proteins (isoforms or quantification standards of the invention), are measured and graphed, which then allows the same properties to be determined for unknown samples (problem samples) by interpolation on the graph. The samples with known properties are the quantification standards, and the graph is the standard curve.

Since the dilutions of the isoforms comprised in the kit of the invention contain a known amount of each one of the beta-conglutin allergen proteins, it is possible to graphically or schematically represent the amount of protein in each of these standard samples versus the absorbance or signal intensity measured in each of these standard samples in step (f). This representation is the "standard curve". An example of standard curve would be one in which the amount of protein, preferably expressed in ng, versus absorbance, preferably at 450 nm, is represented. Preferably, amount of protein is represented in the X axis and absorbance is represented in the Y axis.

Comparison of step (h) allows determining the amount of lupin beta-conglutin allergen proteins in the problem sample by extrapolating the signal intensity results obtained in step (c) to the standard curve previously generated in step (g) with the signal intensity results obtained from the quantification standards in step (f).

Optionally, the method of the invention may further comprise one or more additional steps. An optional additional step would be a step previous to step (a) for the extraction of lupin allergen proteins from problem samples, preferably for the extraction of lupin beta-conglutin allergen proteins. This "extraction step" preferably comprises the homogenization of the isolated sample, for instance by extrution, grinding and/or chopping or similar methods, and the centrifugation of the extract obtained after homogenization. The homogenization may be manual or mechanical, preferably mechanical. More preferably, before the homogenization step the sample is contacted with an extraction buffer. The preferred extraction buffer is that previously described in the present invention. The homogenization step is performed during, preferably, between 1h and 30min and 2h and 30min, more preferably at 4°C. Afterwards, the extract obtained is centrifuged during preferably between 15 and 30 min, more preferably between 12,000 and 17,000 xg, even more preferably at 4°C. Inventors have optimized this protocol for lupin beta-conglutin allergen proteins extraction in order to ensure that these allergen proteins remain in the sample to be tested in case they are present.

For liquid problem samples the extraction step could be as that disclosed in the paragraph above but omitting the homogenization step.

Another optional additional step, which could be performed in the absence or in the presence of the additional step described in the two paragraphs above, would be a step previous to step (a) and previous to step (d) for the attachment (or coating) of the antibody of the invention or the problem and/or standard samples to the support in which the subsequent incubation step will take place. This step could be also called "coating step". This step comprises the incubation of the antibody of the invention or the samples with the support during an adequate period of time to ensure the attachment. After the coating step takes place, the support may be washed one or more times, preferably three times, with a washing solution. The preferred washing solution is that previously described in the present invention. Afterwards, the washing solution is removed.

Another optional additional step, which could be performed in the absence or in the presence of the two additional steps described in the paragraphs above, would be a step for blocking the remaining protein-binding sites after the incubation steps (b) and (e). Preferred blocking solutions to be added for this step are those previously described in the present invention. More preferably, this blocking step takes place during 1 to 2 hours at RT or during 12 hours at 4°C. After the blocking step is completed, a washing step may be performed as described above.

If the ELISA method selected for the detection and/or quantification steps of the present invention requires the use of a secondary antibody, which is for instance the case of an indirect ELISA, an additional step would comprise a second incubation, after steps (b) and (e), with this secondary antibody. These second incubation is preferably performed during 1h and 30 min at RT or 12 hours at 4°C. Afterwards, a washing step may be carried out as explained above.

Another additional step, after the incubation steps, may be performed which comprises the addition of the substrate required for the labeling reaction. This substrate will be the specific for the conjugation system selected. Thus, for instance if the detection or conjugation system selected is HRP, the substrate to be added in this step is TMB. The incubation with this substrate takes place, preferably, during 5 min to 30 min at RT (signal development time can range between 5 to 30 minutes, depending on the assay and on the method used to stop the reaction). Afterwards, this labeling reaction may be stopped by adding an adequate stop solution, preferably sulphuric acid, during more preferably 10 min.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the scope of the present invention.

### DESCRIPTION OF THE DRAWINGS

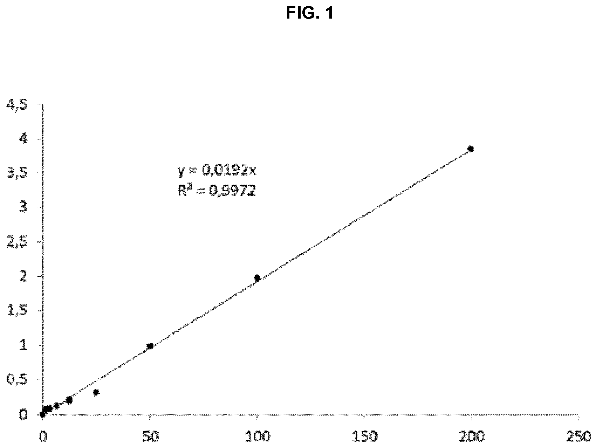

**Fig. 1****. Shows a representative standard curve.** y= Absorbance, x= ng of proteins. y= ax + b; x= (y-b)/x.

### EXAMPLES

### EXAMPLE 1. ANTIBODY PREPARATION.

A peptide was synthetized (Agrisera, Sweeden) with the following sequence "VDEGEGNYELVGIR" (SEQ ID NO: 1) corresponding to an epitope that is present in the allergen Lup an 1 (β1-conglutin) and the six other isoform/variants of the β-congiutin proteins currently identified in *Lupinus angustifolius,* and in the allergen Lup a 1 (β-conglutin) currently identified in *Lupinus albus*. This peptide was used to immunize rabbits and to produce a specific polyclonal antiserum. The rabbit immune serum was affinity-purified against the same synthetic peptide in order to obtain the antibody of the invention specific for the peptide of the invention.

### EXAMPLE 2. ELISA TEST FOR DETECTION AND QUANTIFICATION OF LUPIN ALLERGEN PROTEINS IN LUPIN-CONTAINING FOOD AND BIOLOGICAL SAMPLES.

### A) To Make the Standards for Allergen Proteins Detection and Quantification.

### Coating on well

Coating solution contained: 1a) anti-6xHis Tag antibody of the invention (dilution 1:1000); or 1b) conglutin β1 purified protein at different concentrations (ng) as described in table 1. 1a) or 1b) was used for coating wells. Both alternatives produced comparable results.

The coating solution was removed and wells were washed three times by filling the wells with 200 µL PBS. The solutions or washes were removed by flicking the plate over a sink. The remaining drops were removed by patting the plate on a paper towel.

### Purified conglutin β1 protein to make the standards

If using the coating solution 1a), now must follow the incubation with 1b): 100 µL of diluted purified conglutins β1 (as standards described in table 1) were added to each well. Standards (triplicates) and blank were run with each plate. Incubation was performed overnight at 4°C.

It was ensured that the concentration of standards spans the most dynamic detection range of antibody binding.

The plate was washed three times with 200 µlL of PBS.

### Blocking and washing

The remaining protein-binding sites were blocked in the coated wells by adding 200 µL of blocking buffer (5% non-fat dry milk/PBS) per well, and incubation was performed for 2 h at room temperature (RT).

The plate was washed three times with 200 µL PBS.

The results of standards quantification are shown in Fig. 1 and Table 1.

**Table 1. Results of absorbance in standard samples.**

| Standard Sample | β1 Protein (ng) | Absorbance (450nm) |
|---|---|---|
| 1 | 0 | 0 |
| 2 | 1.55 | 0.069 |
| 3 | 3.10 | 0.081 |
| 4 | 6.30 | 0.119 |
| 5 | 12.50 | 0.193 |
| 6 | 25.00 | 0.305 |
| 7 | 50.00 | 0.978 |
| 8 | 100.00 | 1.971 |
| 9 | 200 | 3.84 |

### B) To Make the Food Allergen Proteins Detection and Quantification Plates coating with food allergen proteins

50 µL of total protein extracts from different foods were mixed with 50 µL of PBS (diluent buffer) and added to the corresponding well to have a 100 µL of total incubation volume.

Incubation was performed for 2h at room temperature (RT). Afterwards, the plate was washed three times with 200 µL of PBS.

### Blocking and washing

The remaining protein-binding sites were blocked in the coated wells by adding 200 µL of blocking buffer (5% non-fat dry milk/PBS) per well, and incubation was performed for 2 h at room temperature (RT). The plate was washed three times with 200 µL PBS.

### Plates coated with proteins (conglutin β1 o food allergen proteins) were incubated with primary antibody

Specific antibody (dilution 1:1000) against β-congiutin proteins (antibody of the invention), without conjugation to an enzyme, was incubated overnight at 4°C. Not linked antibodies were removed by plate well washing three times with 200 µL PBS.

### Incubation with secondary antibody

An anti-Rabbit IgG HRP conjugated antibody was used against the antibody of the invention. Incubations were developed for 1h and 30min at RT. Not linked antibodies were removed by plate well washing three times with 200 µL PBS.

### Signal development

Signal was developed using a compatible substrate for colorimetry, chemiluminiscence, fluorescence, i.e. TMB substrate.

100µL of TMB were added to each well and incubated for 5 min at RT, and the reaction was stopped using 1M Sulphuric Acid for 10 min.

Signal was read in a microplate reader iMak (Bio-Rad) at 450nm. Results are shown in Table 2.

**Table 2. Detection and quantification of the lupin β-congiutin allergen proteins in food and biological samples. ¹Triplicated experiments. ²Allergens β-conglutin proteins quantity corresponds to samples diluted ¼. ³Protein quantity (ng of proteins) was determined in total volume = 200 µL.**

| Food/Biological Samples | Absorbance (450nm)¹ | Allergen β-conglutin proteins quantity² (ng) | Final allergen β-conglutin proteins quantity (ng) | Allergen β-conglutin proteins content (ng/µL or ppm)³ |
|---|---|---|---|---|
| 1 | 0.039 | 2.03125 | 8.125 | 0.040625 |
| | 0.038 | 1.97917 | | |
| | 0.04 | 2.08334 | | |
| 2 | 0 | 0 | 0 | 0 |
| | 0 | 0 | | |
| | 0 | 0 | | |
| 3 | 1.843 | 95.9896 | 391.875 | 1.959375 |
| | 1.843 | 95.9896 | | |
| | 1.957 | 101.9271 | | |
| 4 | 0 | 0 | 0 | 0 |
| | 0 | 0 | | |
| | 0 | 0 | | |
| 5 | 0.389 | 20.2604 | 80.9028 | 0.404514 |
| | 0.386 | 20.1042 | | |
| | 0.39 | 20.3125 | | |
| 6 | 1.343 | 69.9479 | 299.3749 | 1.496875 |
| | 1.567 | 81.6145 | | |
| | 1.401 | 72.9688 | | |
| 7 | 2.289 | 119.2188 | 457.8474 | 2.289237 |
| | 2.103 | 109.5313 | | |
| | 2.201 | 114.6354 | | |
| 8 | 2.894 | 150.7292 | 574.7918 | 2.873959 |
| | 2.691 | 140.1563 | | |
| | 2.692 | 140.2083 | | |
| 9 | 0.019 | 0.9896 | 3.8890 | 0.019445 |
| | 0.02 | 1.0417 | | |
| | 0.017 | 0.8854 | | |
| 10 | 1.034 | 53.8542 | 216.1112 | 1.080556 |
| | 1.038 | 54.0625 | | |
| | 1.04 | 54.1667 | | |
| 11 | 0 | 0 | 0 | 0 |
| | 0 | 0 | | |
| | 0 | 0 | | |
| 12 | 0.784 | 40.8334 | 152.9862 | 0.764931 |
| | 0.706 | 36.7708 | | |
| | 0.713 | 37.1354 | | |
| 13 | 0.643 | 33.4896 | 129.1667 | 0.645834 |
| | 0.600 | 31.2500 | | |
| | 0.617 | 32.1354 | | |
| 14 | 2.903 | 151.1979 | 599.5833 | 2.997917 |
| | 2.845 | 148.1771 | | |
| | 2.886 | 150.3125 | | |
| 15 | 0.284 | 14.7917 | | 0.299331 |
| | 0.293 | 15.2604 | 59.8660 | |
| | 0.285 | 14.8438 | | |
| 16 | 0.019 | 0.9896 | | 0.017709 |
| | 0.017 | 0.8854 | 3.5417 | |
| | 0.015 | 0.7813 | | |

### C) Method for total protein extraction, including allergen proteins, in food and biological samples

Samples were firstly made homogeneous by grinding/chopping. The extract was centrifuged or allowed to settle prior to performing the ELISA method.

Well plates were coated with the above protein extracts, following the methodology previously described.

### Tissue extract

1- The tissue of interest was dissect with clean tools, preferably on ice and as quickly as possible to prevent degradation by proteases.
2- The tissue was placed in round bottom microfuge tubes and immerse in liquid nitrogen to "snap freeze". Samples were stored at -80°C for later use or kept on ice for immediate homogenization.
3- For a ∼5 mg piece of tissue, -300 µL of complete extraction buffer were added to the tube and homogenized with an electric homogenizer.
4- The blade was rinsed twice using 1mL of complete extraction buffer for each rinse, then constant agitation was maintained for 2 h at 4°C for protein extraction.
5- The mixture was centrifuged for 20 min at 15,000 x g at 4°C, placed on ice, aliquoted supernatant (this is the soluble protein extract) to a fresh, chilled tube and samples were stored at -80°C. Freeze/thaw cycles were minimized.

### Milk

Samples were collected and centrifuged at 10,000 x g for 2 min at 4°C. Supernatant was aliquoted and samples were stored at -80°C. Freeze/thaw cycles were minimized.

### Composition of the proteins extraction buffer

100 mM Tris, pH 7.4
150 mM NaCl
1 mM EGTA
1 mM EDTA
1% Triton X-100
0.5% Sodium deoxycholate
Additional reagents required to produce complete extraction buffer:
Protease inhibitor cocktail
PMSF to 1mM, immediately before use.

### D) Food and biological samples used for lupin allergen detection and quantification

1 - Toasted bread, brand: Schär
   Product information: Lupin protein
2 - Gluten Free Maxi Sorrisi Chocolate Biscuit, brand: Schär
   Product information: It may content lupin trazes
3 - Commercial Lupin Flour, brand: Arche Naturküche
   Product information: Sweet lupine flour
4 - Peanut butter, brand: whole earth
   Product information: Roasted peanuts (97%), Palm oil, Sea salt
5 - Carob spread hazelnut Chocolate duo, brand: Carobella organic
   Product information: lupin flour 5%
6 - Seeds (*Lupinus albus* L.)
7 - Seeds (*Lupinus luteus* L.)
8 - Seeds (*Lupinus angustifolius* L.)
9 - Lupinen BOLOGNESE SAUCE, brand: Alberts
   Product information: sweet lupine seeds cooked (8%)
10 - Fresh spread cheese, brand: Made with LUVE
   Product information: lupin protein (6.6%)
11 - Wheat toasted bread, brand: BIMBO
   Product information: non lupin content
12 - Lupinen - Tempeh LUPEH, brand: Alberts
   Product information: Boiled sweet lupin seeds 99%
13 - LupinenBURGER - MEDITERRANEAN, brand: Alberts
   Product information: Boiled sweet lupin seeds 15%
14 - Pickled lupine, brand: Hacendado (Mercadona)
   Product information: Lupin (*L. albus*)*,* salt, lactic acid, citric acid, ascorbic acid, potassium sorbate
15 - Lupinen Drink (total proteins extraction according to proteins extraction methodology), brand: Made with LUVE
   Product information: Lupin protein (2.3%)
16 - Raw Lupinen Drink, brand: Made with LUVE
   Product information: Lupin protein (2.3%)

## Claims

1. An amino acid sequence consisting of SEQ ID NO: 1.

2. A nucleic acid sequence encoding the amino acid sequence according to claim 1.

3. An antibody that specifically binds the amino acid sequence according to claim 1.

4. The antibody according to claim 3 which is conjugated with a detection system.

5. The antibody according to claim 4, wherein the detection system is a dye, fluorochrome or enzyme.

6. A kit comprising the antibody according to any of claims 3 to 5 and the following polypeptides:
a. the beta-conglutin isoforms comprising SEQ ID NO: 2, SEQ ID NO: 3, SEQ ID NO: 4, SEQ ID NO: 5, SEQ ID NO: 6, SEQ ID NO: 7 and SEQ ID NO: 8, and/or
b. the beta-conglutin isoform comprising SEQ ID NO: 9.

7. Use of the antibody according to any of claims 3 to 5 or the kit according to claim 6 for the *in vitro* detection and/or quantification of the lupin beta-conglutin allergen proteins.

8. Use according to claim 7 for the *in vitro* detection and/or quantification of the lupin beta-conglutin allergen proteins in food samples.

9. Use according to any of claims 7 or 8, wherein the lupin beta-conglutin allergen proteins are those comprising SEQ ID NO: 2, SEQ ID NO: 3, SEQ ID NO: 4, SEQ ID NO: 5, SEQ ID NO: 6, SEQ ID NO: 7, SEQ ID NO: 8, and/or SEQ ID NO: 9.

10. An *in vitro* method for the detection and/or quantification of the lupin beta-conglutin allergen proteins in isolated samples, wherein said method comprises the following steps:
a. putting in contact the isolated sample with the antibody according to any of claims 3 to 5 or the kit according to claim 6,
b. incubating the mixture of step (a) under conditions that allow the binding of the antibody to its antigen, and
c. detecting and/or quantifying the binding of step (b).

11. The method according to claim 10, wherein the isolated sample is a food sample.

12. The method according to any of claims 10 or 11, wherein the lupin beta-conglutin allergen proteins are those comprising SEQ ID NO: 2, SEQ ID NO: 3, SEQ ID NO: 4, SEQ ID NO: 5, SEQ ID NO: 6, SEQ ID NO: 7, SEQ ID NO: 8, and/or SEQ ID NO: 9.

13. The method according to any of claims 10 to 12, wherein the detection and/or quantification of step (c) is performed by immunoassay.

14. The method according to claim 13, wherein the immunoassay is ELISA or Western blot, preferably ELISA.

15. The method according to any of claims 10 to 14, which further comprises the following steps:
d. putting in contact different dilutions of each of the isoforms comprised in the kit according to claim 6 with the antibody according to any of claims 3 to 5,
e. incubating the mixture of step (d) under conditions that allow the binding of the antibody to its antigen,
f. detecting and/or quantifying the binding of step (e),
g. generating an standard curve with the detection and/or quantification values obtained in step (f), and
h. comparing the detection and/or quantification values obtained in step (c) with the standard curve generated in step (g).


SEQUENCE LISTING

<110>  Consejo Superior de Investigaciones Científicas (CSIC)

<120>  METHOD FOR THE DETECTION AND/OR QUANTIFICATION OF LUPIN
       BETA-CONGLUTIN ALLERGEN PROTEINS

<130>  EP1641.1299

<160>  9

<170>  PatentIn version 3.5

<210>  1
<211>  14
<212>  PRT
<213>  Artificial Sequence

<220>
<223>  Epitope of the invention

<400>  1

Val Asp Glu Gly Glu Gly Asn Tyr Glu Leu Val Gly Ile Arg
1               5                   10


<210>  2
<211>  611
<212>  PRT
<213>  Lupinus angustifolius

<400>  2

Met Ala Lys Met Arg Val Arg Leu Pro Met Leu Ile Leu Leu Leu Gly
1               5                   10                  15


Val Val Phe Leu Leu Ala Ala Ser Ile Gly Ile Ala Tyr Gly Glu Lys
            20                  25                  30


Asp Phe Thr Lys Asn Pro Pro Lys Glu Arg Glu Glu Glu Glu His Glu
        35                  40                  45


Pro Arg Gln Gln Pro Arg Pro Arg Gln Gln Glu Glu Gln Glu Arg Glu
    50                  55                  60


His Arg Arg Glu Glu Lys His Asp Gly Glu Pro Ser Arg Gly Arg Ser
65                  70                  75                  80


Gln Ser Glu Glu Ser Gln Glu Glu Glu His Glu Arg Arg Arg Glu His
                85                  90                  95


His Arg Glu Arg Glu Gln Glu Gln Gln Pro Arg Pro Gln Arg Arg Gln
            100                 105                 110


Glu Glu Glu Glu Glu Glu Glu Glu Trp Gln Pro Arg Arg Gln Arg Pro

115                 120                 125

Gln Ser Arg Arg Glu Glu Arg Glu Glu Arg Glu Gln Glu Gln Gly Ser
    130                 135                 140

Ser Ser Gly Ser Gln Arg Gly Ser Gly Asp Glu Arg Arg Gln His Arg
145                 150                 155                 160

Glu Arg Arg Val His Arg Glu Glu Arg Glu Gln Glu Gln Asp Ser Arg
                165                 170                 175

Ser Asp Ser Arg Arg Gln Arg Asn Pro Tyr His Phe Ser Ser Asn Arg
            180                 185                 190

Phe Gln Thr Tyr Tyr Arg Asn Arg Asn Gly Gln Ile Arg Val Leu Glu
        195                 200                 205

Arg Phe Asn Gln Arg Thr Asn Arg Leu Glu Asn Leu Gln Asn Tyr Arg
    210                 215                 220

Ile Ile Glu Phe Gln Ser Lys Pro Asn Thr Leu Ile Leu Pro Lys His
225                 230                 235                 240

Ser Asp Ala Asp Phe Ile Leu Val Val Leu Asn Gly Arg Ala Thr Ile
                245                 250                 255

Thr Ile Val Asn Pro Asp Lys Arg Gln Val Tyr Asn Leu Glu Gln Gly
            260                 265                 270

Asp Ala Leu Arg Leu Pro Ala Gly Thr Thr Ser Tyr Ile Leu Asn Pro
        275                 280                 285

Asp Asp Asn Gln Asn Leu Arg Val Ala Lys Leu Ala Ile Pro Ile Asn
    290                 295                 300

Asn Pro Gly Lys Leu Tyr Asp Phe Tyr Pro Ser Thr Thr Lys Asp Gln
305                 310                 315                 320

Gln Ser Tyr Phe Ser Gly Phe Ser Lys Asn Thr Leu Glu Ala Thr Phe
                325                 330                 335

Asn Thr Arg Tyr Glu Glu Ile Glu Arg Val Leu Leu Gly Asp Asp Glu
            340                 345                 350

Leu Gln Glu Asn Glu Lys Gln Arg Arg Gly Gln Glu Gln Ser His Gln
        355                 360                 365

Asp Glu Gly Val Ile Val Arg Val Ser Lys Lys Gln Ile Gln Glu Leu
    370                 375                 380

Arg Lys His Ala Gln Ser Ser Ser Gly Glu Gly Lys Pro Ser Glu Ser
385                 390                 395                 400

Gly Pro Phe Asn Leu Arg Ser Asn Lys Pro Ile Tyr Ser Asn Lys Phe
                405                 410                 415

Gly Asn Phe Tyr Glu Ile Thr Pro Asp Ile Asn Pro Gln Phe Gln Asp
            420                 425                 430

Leu Asn Ile Ser Leu Thr Phe Thr Glu Ile Asn Glu Gly Ala Leu Leu
        435                 440                 445

Leu Pro His Tyr Asn Ser Lys Ala Ile Phe Ile Val Val Val Asp Glu
    450                 455                 460

Gly Glu Gly Asn Tyr Glu Leu Val Gly Ile Arg Asp Gln Gln Arg Gln
465                 470                 475                 480

Gln Asp Glu Gln Glu Glu Glu Tyr Glu Gln Gly Glu Glu Glu Val Arg
                485                 490                 495

Arg Tyr Ser Asp Lys Leu Ser Lys Gly Asp Val Phe Ile Ile Pro Ala
            500                 505                 510

Gly His Pro Leu Ser Ile Asn Ala Ser Ser Asn Leu Arg Leu Leu Gly
        515                 520                 525

Phe Gly Ile Asn Ala Asn Glu Asn Gln Arg Asn Phe Leu Ala Gly Ser
    530                 535                 540

Glu Asp Asn Val Ile Lys Gln Leu Asp Arg Glu Val Lys Glu Leu Thr
545                 550                 555                 560

Phe Pro Gly Ser Ile Glu Asp Val Glu Arg Leu Ile Lys Asn Gln Gln
                565                 570                 575

Gln Ser Tyr Phe Ala Asn Ala Gln Pro Gln Gln Gln Gln Gln Arg Glu
            580                 585                 590

Lys Glu Gly Arg Arg Gly Arg Arg Gly Pro Ile Ser Ser Ile Leu Asn
        595                 600                 605

Ala Leu Tyr
    610

<210>  3
<211>  603
<212>  PRT
<213>  Lupinus angustifolius

<400>  3

Met Ala Asn Met Arg Val Lys Phe Pro Thr Leu Val Leu Leu Leu Gly
1               5                   10                  15

Ile Val Phe Leu Met Ala Val Ser Ile Gly Ile Ala Tyr Gly Glu Lys
            20                  25                  30

Asn Ala Ile Lys Asn His Glu Arg Pro Gln Glu Arg Glu Gln Glu Glu
        35                  40                  45

Arg Asp Pro Arg Gln Gln Pro Arg Pro Arg His Gln Glu Glu Gln Glu
    50                  55                  60

Arg Glu His Gly Arg Glu Glu Glu Arg Asn Arg Glu Pro Ser Arg Gly
65                  70                  75                  80

Arg Ser Glu Ser Glu Glu Ser Arg Glu Glu Glu Arg Glu Gln Arg Arg
                85                  90                  95

Glu Pro Ser Arg Gly Arg Glu Gln Glu Gln Gln Pro Gln His Gly Arg
            100                 105                 110

Arg Glu Glu Glu Glu Glu Trp Gln Pro Arg Arg Gln Arg Pro Gln Ser
        115                 120                 125

Arg Arg Glu Glu Arg Glu Gln Glu Gln Gly Ser Ser Ser Ser Ser Gly
    130                 135                 140

Arg Gln Ser Gly Tyr Glu Arg Arg Glu Gln Arg Glu Glu Arg Glu Gln
145                 150                 155                 160

Gln Gln Glu Gln Asp Ser Arg Ser Glu Ser Arg Arg Gln Arg Asn Pro
                165                 170                 175

Tyr Tyr Phe Ser Tyr Glu Arg Phe Gln Thr Leu Tyr Lys Asn Arg Asn
            180                 185                 190

Gly Gln Ile Arg Val Leu Glu Arg Phe Asp Gln Arg Thr Asn Arg Leu
        195                 200                 205

Glu Asn Leu Gln Asn Tyr Arg Ile Val Glu Phe Gln Ser Lys Pro Asn
    210                 215                 220

Thr Leu Ile Leu Pro Lys His Ser Asp Ala Asp Tyr Ile Leu Val Val
225                 230                 235                 240

Leu Asn Gly Arg Ala Thr Ile Thr Ile Val Asn Pro Asp Lys Arg Gln
                245                 250                 255

Ala Tyr Asn Leu Glu His Gly Asp Ala Leu Arg Leu Pro Ala Gly Thr
            260                 265                 270

Thr Ser Tyr Ile Leu Asn Pro Asp Asp Asn Gln Asn Leu Arg Val Val
        275                 280                 285

Lys Leu Ala Ile Pro Ile Asn Asn Pro Gly Asn Phe Tyr Asp Phe Tyr
    290                 295                 300

Pro Ser Ser Thr Lys Asp Gln Gln Ser Tyr Phe Asn Gly Phe Ser Arg
305                 310                 315                 320

Asn Thr Leu Glu Ala Thr Phe Asn Thr Arg Tyr Glu Glu Ile Gln Arg
                325                 330                 335

Ile Ile Leu Gly Asn Glu Asp Gly Gln Glu Asp Glu Glu Gln Ser Arg
            340                 345                 350

Gly Gln Glu Gln Ser His Gln Asp Gln Gly Val Ile Val Arg Val Ser
        355                 360                 365

Lys Glu Gln Ile Gln Glu Leu Arg Lys His Ala Gln Ser Ser Ser Gly
    370                 375                 380

Lys Gly Lys Pro Ser Glu Ser Gly Pro Phe Asn Leu Arg Ser Asp Glu
385                 390                 395                 400

Pro Ile Tyr Ser Asn Lys Phe Gly Asn Phe Tyr Glu Ile Thr Pro Asp
                405                 410                 415

Arg Asn Pro Gln Ala Gln Asp Leu Asp Ile Ser Leu Thr Phe Ile Glu
            420                 425                 430

Ile Asn Glu Gly Gly Leu Leu Leu Pro His Tyr Asn Ser Lys Ala Ile
        435                 440                 445

Phe Val Val Val Val Asp Glu Gly Glu Gly Asn Tyr Glu Leu Val Gly
    450                 455                 460

Ile Arg Asp Gln Glu Arg Gln Gln Asp Glu Gln Glu Gln Glu Glu Val
465                 470                 475                 480

Arg Arg Tyr Asn Ala Lys Leu Ser Glu Gly Asp Ile Phe Val Ile Pro
                485                 490                 495

Ala Gly His Pro Ile Ser Ile Asn Ala Ser Ser Asn Leu Arg Leu Leu
            500                 505                 510

Gly Phe Gly Ile Asn Ala Asp Glu Asn Gln Arg Asn Phe Leu Ala Gly
        515                 520                 525

Ser Glu Asp Asn Val Ile Arg Gln Leu Asp Lys Glu Val Lys Gln Leu
    530                 535                 540

Thr Phe Pro Gly Ser Val Glu Asp Val Glu Arg Leu Ile Lys Asn Gln
545                 550                 555                 560

Gln Gln Ser Tyr Phe Ala Asn Ala Gln Pro Gln Gln Gln Gln Gln Arg
                565                 570                 575

Glu Lys Glu Gly Arg Arg Gly Arg Arg Gly Leu Ser Phe Pro Phe Arg
            580                 585                 590

Ser Leu Phe Thr Lys Leu Leu Ser Thr Ile Met
        595                 600

<210>  4
<211>  580
<212>  PRT
<213>  Lupinus angustifolius

<400>  4
   Met Ala Lys Met Arg Val Arg Phe Pro Thr Leu Val Leu Leu Leu Gly
   1               5                   10                  15
   Ile Val Phe Leu Met Ala Val Ser Ile Gly Ile Ala Tyr Gly Glu Lys
               20                  25                  30
   Asn Val Leu Lys Asn His Glu Arg Pro Gln Glu Arg Glu Gln Glu Glu
           35                  40                  45
   Arg Asp Pro Arg Gln Gln Pro Arg Pro His His Gln Glu Glu Gln Glu
       50                  55                  60
   Arg Glu His Arg Arg Glu Ser Glu Glu Ser Gln Glu Glu Glu Arg Glu
   65                  70                  75                  80
   Gln Arg Arg Glu Pro Arg Arg Glu Arg Glu Gln Glu Gln Gln Pro Gln
                   85                  90                  95
   His Gly Arg Arg Glu Glu Glu Glu Glu Trp Gln Pro Arg Arg Gln Arg
   100 105 110
   Pro Gln Ser Arg Arg Glu Glu Arg Glu Gln Glu Gln Gly Ser Ser Ser
   115 120 125
   Ser Ser Arg Arg Gln Ser Gly Tyr Glu Arg Arg Glu Gln Arg Glu Glu
   130 135 140
   Arg Glu Gln Glu Gln Glu Gln Gly Ser Arg Ser Asp Ser Arg Arg Gln
   145 150 155 160
   Arg Asn Pro Tyr Tyr Phe Ser Ser Glu Arg Phe Gln Thr Leu Tyr Arg
   165 170 175
   Asn Arg Asn Gly Gln Ile Arg Val Leu Glu Arg Phe Asp Gln Arg Thr
   180 185 190
   Asn Arg Leu Glu Asn Leu Gln Asn Tyr Arg Ile Val Glu Phe Gln Ser
   195 200 205
   Lys Pro Asn Thr Leu Ile Leu Pro Lys His Ser Asp Ala Asp Tyr Ile
   210 215 220
   Leu Val Val Leu Asn Gly Ser Ala Thr Ile Thr Ile Val Asn Pro Asp
   225 230 235 240
   Lys Arg Gln Ser Tyr Asn Leu Glu Asn Gly Asp Ala Leu Arg Leu Pro
   245 250 255
   Ala Gly Thr Thr Ser Tyr Ile Leu Asn Pro Asp Asp Asn Gln Asn Leu
   260 265 270
   Arg Val Val Lys Leu Ala Ile Pro Ile Asn Asn Pro Gly Asn Phe Tyr
   275 280 285
   Asp Phe Tyr Pro Ser Ser Ser Lys Asp Gln Gln Ser Tyr Phe Ser Gly
   290 295 300
   Phe Ser Lys Asn Thr Leu Glu Ala Thr Phe Asn Thr Arg Tyr Glu Glu
   305 310 315 320
   Ile Gln Ser Ile Leu Leu Gly Asn Glu Asp Glu Gln Glu Asp Asp Glu
   325 330 335
   Gln Trp His Gly Gln Glu Gln Ser His Gln Asp Glu Gly Val Ile Val
   340 345 350
   Arg Val Ser Lys Glu Gln Val Gln Glu Leu Arg Lys Tyr Ala Gln Ser
   355 360 365
   Ser Ser Arg Lys Gly Lys Pro Tyr Glu Ser Gly Pro Phe Asn Leu Arg
   370 375 380
   Ser Asn Lys Pro Ile Tyr Ser Asn Lys Phe Gly Asn Phe Tyr Glu Ile
   385 390 395 400
   Thr Pro Asp Arg Asn Pro Gln Ala Gln Asp Leu Asp Ile Ser Leu Thr
   405 410 415
   Phe Ile Glu Ile Asn Glu Gly Ala Leu Leu Leu Pro His Tyr Asn Ser
   420 425 430
   Lys Ala Ile Phe Val Val Val Val Asp Glu Gly Glu Gly Asn Tyr Glu
   435 440 445
   Leu Val Gly Ile Arg Asp Gln Gln Arg Gln Gln Asp Glu Gln Glu Val
   450 455 460
   Arg Arg Tyr Ser Ala Arg Leu Ser Glu Gly Asp Ile Phe Val Ile Pro
   465 470 475 480
   Ala Gly His Pro Ile Ser Ile Asn Ala Ser Ser Asn Leu Arg Leu Leu
   485 490 495
   Gly Phe Gly Ile Asn Ala Asp Glu Asn Gln Arg Asn Phe Leu Ala Gly
   500 505 510
   Ser Glu Asp Asn Val Ile Arg Gln Leu Asp Arg Glu Val Lys Gly Leu
   515 520 525
   Ile Phe Pro Gly Ser Ala Glu Asp Val Glu Arg Leu Ile Lys Asn Gln
   530 535 540
   Gln Gln Ser Tyr Phe Ala Asn Ala Gln Pro Gln Gln Gln Gln Gln Arg
   545 550 555 560
   Glu Arg Glu Gly Arg His Gly Arg Arg Gly His Ile Ser Ser Ile Leu
   565 570 575
   Ser Thr Leu Tyr
   580

<210>  5
<211>  590
<212>  PRT
<213>  Lupinus angustifolius

<400>  5

Met Ile Lys Met Arg Val Arg Phe Pro Thr Leu Val Leu Leu Leu Gly
1               5                   10                  15

Ile Val Phe Leu Met Ala Val Ser Ile Gly Ile Ala Tyr Gly Glu Lys
            20                  25                  30

Asn Val Ile Lys Asn His Glu Arg Pro Gln Glu Arg Glu Gln Glu Glu
        35                  40                  45

Arg Asp Pro Arg Gln Gln Pro Arg Pro His His Gln Glu Glu Gln Glu
    50                  55                  60

Arg Glu His Arg Arg Glu Glu Glu Arg Asp Arg Glu Pro Ser Arg Gly
65                  70                  75                  80

Arg Ser Glu Ser Glu Glu Ser Arg Glu Glu Glu Arg Glu Gln Arg Arg
                85                  90                  95

Glu Pro Arg Arg Glu Arg Glu Gln Glu Gln Gln Pro Gln His Gly Arg
            100                 105                 110

Arg Glu Glu Glu Glu Glu Trp Gln Pro Arg Arg Gln Arg Pro Gln Ser
        115                 120                 125

Arg Arg Glu Glu Arg Glu Gln Glu Gln Gly Ser Ser Ser Ser Ser Arg
    130                 135                 140

Arg Gln Ser Gly Tyr Glu Arg Arg Glu Glu Arg Glu Gln Glu Gln Glu
145                 150                 155                 160

Gln Gly Ser Arg Ser Asp Ser Arg Arg Gln Arg Asn Pro Tyr Tyr Phe
                165                 170                 175

Ser Ser Glu Arg Phe Gln Thr Leu Tyr Arg Asn Arg Asn Gly Gln Ile
            180                 185                 190

Arg Val Leu Glu Arg Phe Asp Gln Arg Thr Asp Arg Leu Glu Asn Leu
        195                 200                 205

Gln Asn Tyr Arg Ile Val Glu Phe Gln Ser Lys Pro Asn Thr Leu Ile
    210                 215                 220

Leu Pro Lys His Ser Asp Ala Asp Tyr Ile Leu Val Val Leu Asn Gly
225                 230                 235                 240

Ser Ala Thr Ile Thr Ile Val Asn Pro Asp Lys Arg Gln Ser Tyr Asn
                245                 250                 255

Leu Glu Asn Gly Asp Ala Leu Arg Leu Pro Ala Gly Thr Thr Ser Tyr
            260                 265                 270

Ile Leu Asn Pro Asp Asp Asn Gln Asn Leu Arg Val Val Lys Leu Ala
        275                 280                 285

Ile Pro Ile Asn Asn Pro Gly Asn Phe Tyr Asp Phe Tyr Pro Ser Ser
    290                 295                 300

Ser Lys Asp Gln Gln Ser Tyr Phe Ser Gly Phe Ser Arg Asn Thr Leu
305                 310                 315                 320

Glu Ala Thr Phe Asn Thr Arg Tyr Glu Glu Ile Gln Arg Ile Leu Leu
                325                 330                 335

Gly Asn Glu Asp Glu Gln Glu Asp Asp Glu Gln Arg His Gly Gln Glu
            340                 345                 350

Gln Ser His Gln Asp Glu Gly Val Ile Val Arg Val Ser Lys Glu Gln
        355                 360                 365

Val Gln Glu Leu Arg Lys Tyr Ala Gln Ser Ser Ser Arg Lys Gly Lys
    370                 375                 380

Pro Ser Lys Ser Gly Pro Phe Asn Leu Arg Ser Asn Lys Pro Ile Tyr
385                 390                 395                 400

Ser Asn Lys Phe Gly Asn Phe Tyr Glu Ile Thr Pro Asn Arg Asn Pro
                405                 410                 415

Gln Ala Gln Asp Leu Asp Ile Ser Leu Thr Phe Ile Glu Ile Asn Glu
            420                 425                 430

Gly Ala Leu Leu Leu Pro His Tyr Asn Ser Lys Ala Ile Phe Val Val
        435                 440                 445

Leu Val Asp Glu Gly Glu Gly Asn Tyr Glu Leu Val Gly Ile Arg Asp
    450                 455                 460

Gln Gln Arg Gln Gln Asp Glu Gln Glu Val Arg Arg Tyr Ser Ala Arg
465                 470                 475                 480

Leu Ser Glu Gly Asp Ile Phe Val Ile Pro Ala Gly His Pro Ile Ser
                485                 490                 495

Ile Asn Ala Ser Ser Asn Leu Arg Leu Leu Gly Phe Gly Ile Asn Ala
            500                 505                 510

Asp Glu Asn Gln Arg Asn Phe Leu Ala Gly Ser Glu Asp Asn Val Ile
        515                 520                 525

Arg Gln Leu Asp Thr Glu Val Lys Gly Leu Thr Phe Pro Gly Ser Thr
    530                 535                 540

Glu Asp Val Glu Arg Leu Ile Lys Asn Gln Gln Gln Ser Tyr Phe Ala
545                 550                 555                 560

Asn Ala Gln Pro Gln Gln Gln Gln Gln Arg Glu Arg Glu Gly Arg Arg
                565                 570                 575

Gly Arg Arg Gly His Ile Ser Ser Ile Leu Ser Thr Leu Tyr
            580                 585                 590

<210>  6
<211>  637
<212>  PRT
<213>  Lupinus angustifolius

<400>  6
   Met Ala Lys Met Arg Val Arg Phe Pro Met Leu Val Leu Leu Leu Gly
   1               5                   10                  15
   Val Val Phe Leu Leu Ala Val Ser Ile Gly Ile Ala Tyr Gly Glu Lys
               20                  25                  30
   Asp Val Ile Lys Asn Pro Glu Arg Pro Glu Glu Arg Gln Glu Glu Glu
           35                  40                  45
   Arg Asp Pro Arg Gln Pro Pro Arg Ser Arg Gln Gln Glu Glu Gln Glu
       50                  55                  60
   Arg Glu His Arg Arg Glu Lys Glu Arg Asp Arg Glu Pro Ser Arg Gly
   65                  70                  75                  80
   Arg Ser Glu Ser Lys Gln Ser Gln Glu Glu Glu Arg Glu Arg Arg Lys
                   85                  90                  95
   Glu His Asp Arg Glu Arg Glu Gln Glu Gln Gln Pro Gln Tyr Gly Arg
               100                 105                 110
   Arg His Glu Glu Glu Glu Lys Gly Glu Glu Glu Glu Glu Gly Gln Ala
           115                 120                 125
   Arg Arg Gln Arg Pro Gln Arg Arg Arg Glu Glu Arg Glu Gln Glu Gln
       130                 135                 140
   Gly Ser Ser Ser Glu Ser Arg Arg Gln Ser Gly Asp Glu Arg Arg His
   145                 150                 155                 160
   Arg His Glu Lys Arg Glu Gln Arg Glu Glu Arg Glu Gln Glu Gln Gly
                   165                 170                 175
   Ser Ser Ser Gly Arg Gln Ser Asp Tyr Gly Arg Arg Gln Arg His Glu
               180                 185                 190
   Gly Arg Glu Gln Arg Glu Glu Arg Glu Gln Glu Gln Gly Ser Ser Ser
           195                 200                 205
   Glu Ser His Arg Leu Arg Asn Pro Tyr Tyr Phe Ser Ser Glu Arg Phe
       210                 215                 220
   Gln Thr Arg Tyr Lys Asn Lys Asn Gly Gln Ile Arg Val Leu Glu Arg
   225                 230                 235                 240
   Phe Asp Gln Arg Thr Asn Arg Leu Glu Asn Leu Gln Asn Tyr Arg Ile
                   245                 250                 255
   Val Glu Phe Gln Ser Arg Pro Asn Thr Leu Ile Leu Pro Lys His Ser
               260                 265                 270
   Asp Ala Asp Tyr Ile Leu Val Val Leu Asn Gly Arg Ala Thr Ile Thr
           275                 280                 285
   Ile Val Asn Pro Asp Lys Arg Gln Ala Tyr Asn Leu Glu Tyr Gly Asp
       290                 295                 300
   Ala Leu Arg Leu Pro Ala Gly Thr Thr Ser Tyr Ile Leu Asn Pro Asp
   305                 310                 315                 320
   Asp Asn Gln Asp Leu Arg Val Val Lys Leu Ala Ile Pro Ile Asn Asn
                   325                 330                 335
   Pro Gly Lys Phe Tyr Asp Phe Tyr Pro Ser Arg Thr Lys Asp Gln Gln
               340                 345                 350
   Ser Tyr Phe Ser Gly Phe Ser Lys Asn Thr Leu Glu Ala Thr Phe Asn
           355                 360                 365
   Thr His Tyr Glu Glu Ile Gln Arg Ile Leu Leu Gly Tyr Glu Asp Glu
       370                 375                 380
   Gln Glu Asp Glu Glu Gln Arg Arg Glu Gln Glu Gln Ser His Gln Asp
   385                 390                 395                 400
   Glu Gly Val Ile Val Arg Val Ser Lys Glu Gln Ile Gln Glu Leu Arg
                   405                 410                 415
   Lys His Ala Gln Ser Ser Ser Arg Lys Gly Lys Pro Ser Glu Ser Gly
               420                 425                 430
   Pro Phe Asn Leu Arg Ser Asn Glu Pro Ile Tyr Ser Asn Lys Phe Gly
           435                 440                 445
   Asn Phe Tyr Glu Ile Thr Pro Asp Arg Asn Pro Gln Val Gln Asp Leu
       450                 455                 460
   Asp Ile Ser Leu Ile Phe Thr Glu Ile Ser Glu Gly Ala Leu Leu Leu
   465                 470                 475                 480
   Pro His Tyr Asn Ser Lys Ala Ile Phe Val Ile Val Val Asp Glu Gly
                   485                 490                 495
   Glu Gly Asn Tyr Glu Leu Val Gly Ile Arg Asn Gln Gln Arg Gln Gln
               500                 505                 510
   Asp Glu Gln Glu Val Glu Glu Val Arg Ser Tyr Asn Ala Arg Leu Ser
           515                 520                 525
   Glu Gly Asp Ile Leu Val Ile Pro Ala Gly His Pro Leu Ser Ile Asn
       530                 535                 540
   Ala Ser Ser Asn Leu Arg Leu Leu Gly Phe Gly Ile Asn Ala Asp Glu
   545                 550                 555                 560
   Asn Gln Arg Asn Phe Leu Ala Gly Ser Glu Asp Asn Val Ile Arg Gln
                   565                 570                 575
   Leu Asp Arg Glu Val Lys Glu Leu Ile Phe Pro Gly Ser Ala Glu Asp
               580                 585                 590
   Val Glu Arg Leu Ile Arg Asn Gln Gln Gln Ser Tyr Phe Ala Asn Ala
           595                 600                 605
   Gln Pro Gln Gln Gln Gln Gln Gln Arg Glu Lys Glu Gly Arg Arg Gly
   610                 615                 620
   Arg Arg Gly Pro Ile Ser Ser Ile Leu Ser Ala Leu Tyr
   625                 630                 635

<210>  7
<211>  593
<212>  PRT
<213>  Lupinus angustifolius

<400>  7

Met Ile Lys Met Arg Val Arg Phe Pro Thr Leu Val Leu Leu Leu Gly
1               5                   10                  15

Ile Val Phe Leu Met Ala Val Ser Ile Gly Ile Ala Tyr Gly Glu Lys
            20                  25                  30

Asn Val Ile Lys Asn His Glu Arg Pro Gln Glu Arg Glu Gln Glu Glu
        35                  40                  45

Arg Asp Pro Arg Gln Gln Pro Arg Pro His His Gln Glu Glu Gln Glu
    50                  55                  60

Arg Glu His Arg Arg Glu Glu Glu Arg Asp Arg Glu Pro Ser Arg Gly
65                  70                  75                  80

Arg Arg Glu Ser Glu Glu Ser Arg Glu Glu Glu Arg Glu Gln Arg Arg
                85                  90                  95

Glu Pro Arg Arg Glu Arg Glu Gln Glu Gln Gln Pro Gln His Gly Arg
            100                 105                 110

Arg Glu Glu Glu Glu Glu Trp Gln Pro Arg Arg Gln Arg Pro Gln Ser
        115                 120                 125

Arg Arg Glu Glu Arg Glu Gln Glu Gln Gly Ser Ser Ser Ser Ser Arg
    130                 135                 140

Arg Gln Ser Ala Tyr Glu Arg Arg Glu Gln Arg Glu Glu Arg Glu Gln
145                 150                 155                 160

Glu Gln Glu Gln Gly Ser Arg Ser Asp Ser Arg Arg Gln Arg Asn Pro
                165                 170                 175

Tyr Tyr Phe Ser Ser Glu Arg Phe Gln Thr Leu Tyr Arg Asn Arg Asn
            180                 185                 190

Gly Gln Ile Arg Val Leu Glu Arg Phe Asp Lys Arg Thr Asp Arg Leu

195                 200                 205

Glu Asn Leu Gln Asn Tyr Arg Ile Val Glu Phe Gln Ser Lys Pro Asn
    210                 215                 220

Thr Leu Ile Leu Pro Lys His Ser Asp Ala Asp Tyr Ile Leu Val Val
225                 230                 235                 240

Leu Asn Gly Ser Ala Thr Ile Thr Ile Val Asn Pro Asp Lys Arg Gln
                245                 250                 255

Ser Tyr Asn Leu Glu Asn Gly Asp Ala Leu Arg Leu Pro Ala Gly Thr
            260                 265                 270

Thr Ser Tyr Ile Leu Asn Pro Asp Asp Asn Gln Asn Leu Arg Val Val
        275                 280                 285

Lys Leu Ala Ile Pro Ile Asn Asn Pro Gly Asn Phe Tyr Asp Phe Tyr
    290                 295                 300

Pro Ser Ser Ser Lys Asp Gln Gln Ser Tyr Phe Ser Gly Phe Ser Arg
305                 310                 315                 320

Asn Thr Leu Glu Ala Thr Phe Asn Thr Arg Tyr Glu Glu Ile Gln Arg
                325                 330                 335

Ile Leu Leu Gly Asn Glu Asp Glu Gln Glu Asp Asp Glu Gln Arg His
            340                 345                 350

Gly Gln Glu Gln Ser His Gln Asp Glu Gly Val Ile Val Arg Val Ser
        355                 360                 365

Lys Glu Gln Val Gln Glu Leu Arg Lys Tyr Ala Gln Ser Ser Ser Arg
    370                 375                 380

Lys Gly Lys Pro Ser Lys Ser Gly Pro Phe Asn Leu Arg Ser Asn Lys
385                 390                 395                 400

Pro Ile Tyr Ser Asn Lys Phe Gly Asn Phe Tyr Glu Ile Thr Pro Asn
                405                 410                 415

Arg Asn Pro Gln Ala Gln Asp Leu Asp Ile Ser Leu Thr Phe Ile Glu
            420                 425                 430

Ile Asn Glu Gly Ala Leu Leu Leu Pro His Tyr Asn Ser Lys Ala Ile
        435                 440                 445

Phe Val Val Leu Val Asp Glu Gly Glu Gly Asn Tyr Glu Leu Val Gly
    450                 455                 460

Ile Arg Asp Gln Gln Arg Gln Gln Asp Glu Gln Glu Val Arg Arg Tyr
465                 470                 475                 480

Ser Ala Arg Leu Ser Glu Gly Asp Ile Phe Val Ile Pro Ala Gly His
                485                 490                 495

Pro Ile Ser Ile Asn Ala Ser Ser Asn Phe Arg Leu Leu Gly Phe Gly
            500                 505                 510

Ile Asn Ala Asp Glu Asn Gln Arg Asn Phe Leu Ala Gly Phe Glu Asp
        515                 520                 525

Asn Val Ile Arg Gln Leu Asp Arg Glu Val Lys Gly Leu Thr Phe Pro
    530                 535                 540

Gly Phe Ala Glu Asp Val Glu Arg Leu Ile Lys Asn Gln Gln Gln Ser
545                 550                 555                 560

Tyr Phe Ala Asn Ala Gln Pro Gln Gln Gln Gln Gln Arg Glu Arg Glu
                565                 570                 575

Gly Arg His Gly Arg Arg Gly His Ile Phe Ser Ile Leu Ser Thr Leu
            580                 585                 590

Tyr

<210>  8
<211>  605
<212>  PRT
<213>  Lupinus angustifolius

<400>  8

Met Ala Arg Met Arg Val Arg Phe Pro Thr Leu Val Leu Leu Leu Gly
1               5                   10                  15

Ile Leu Phe Leu Met Ala Val Ser Ile Gly Ile Ala Tyr Gly Glu Lys
            20                  25                  30

Asp Val Ile Lys Asn His Glu Arg Pro Gly Glu Arg Glu His Glu Glu
        35                  40                  45

Arg Asp Pro Arg Gln Gln Pro Arg Pro Arg Lys Gln Glu Glu Gln Glu
    50                  55                  60

Arg Glu His Arg Arg Glu Glu Glu His Asp Arg Asp Pro Ser Arg Gly
65                  70                  75                  80

Arg Arg Glu Ser Glu Glu Arg Gln Glu Glu Glu Arg Glu Arg Arg Arg
                85                  90                  95

Glu Pro Cys Arg Glu Arg Glu Gln Glu Gln Gln Pro Gln His Gly Arg
            100                 105                 110

Arg Glu Glu Glu Glu Glu Glu Glu Glu Trp Gln Pro Arg Arg Leu Arg
        115                 120                 125

Pro Gln Ser Arg Lys Glu Glu Arg Glu Gln Glu Gln Gly Ser Ser Ser
    130                 135                 140

Ser Ser Arg Lys Gln Ser Gly Tyr Glu Arg Arg Gln Tyr His Glu Arg
145                 150                 155                 160

Arg Glu Gln Arg Asp Glu Lys Glu Lys Glu Gln Asp Ser Arg Ser Asp
                165                 170                 175

Ser Arg Arg Gln Arg Asn Pro Tyr His Phe Ser Ser Glu Arg Phe Gln
            180                 185                 190

Thr Arg Tyr Arg Asn Arg Asn Gly Gln Ile Arg Val Leu Glu Arg Phe
        195                 200                 205

Asp Gln Arg Thr Asn Arg Leu Glu Asn Leu Gln Asn Tyr Arg Ile Val
    210                 215                 220

Glu Phe Gln Ser Asn Pro Asn Thr Leu Ile Leu Pro Lys His Ser Asp
225                 230                 235                 240

Ala Asp Tyr Ile Leu Val Val Leu Asn Gly Arg Ala Thr Ile Thr Ile
                245                 250                 255

Val Asn Pro Asp Lys Arg Gln Ala Tyr Asn Leu Glu Tyr Gly Asp Ala
            260                 265                 270

Leu Arg Val Pro Ala Gly Thr Thr Ser Tyr Ile Leu Asn Pro Asp Asp
        275                 280                 285

Asn Gln Asn Leu Arg Val Val Lys Leu Ala Ile Pro Ile Asn Asn Pro
    290                 295                 300

Ser Asn Phe Tyr Asp Phe Tyr Pro Ser Ser Thr Lys Asp Gln Gln Ser
305                 310                 315                 320

Tyr Phe Ser Gly Phe Ser Lys Asn Thr Leu Glu Ala Thr Phe Asn Thr
                325                 330                 335

Arg Tyr Glu Glu Ile Gln Arg Ile Leu Leu Gly Asn Glu Asp Glu Gln
            340                 345                 350

Glu Asp Glu Glu Gln Arg Arg Gly Gln Glu Gln Ser Tyr Gln Asp Glu
        355                 360                 365

Gly Val Ile Val Arg Val Ser Lys Glu Gln Ile Gln Glu Leu Arg Lys
    370                 375                 380

His Ala Gln Ser Ser Ser Arg Lys Gly Lys Pro Ser Glu Ser Gly Pro
385                 390                 395                 400

Phe Asn Leu Arg Ser Asn Glu Ser Ile Tyr Ser Asn Lys Phe Gly Asn
                405                 410                 415

Phe Tyr Glu Ile Thr Pro Glu Arg Asn Pro Gln Val Gln Asp Leu Asp
            420                 425                 430

Ile Ser Leu Thr Phe Thr Glu Ile Asn Glu Gly Ala Leu Leu Leu Pro
        435                 440                 445

His Tyr Asn Ser Lys Ala Ile Phe Ile Val Val Val Asp Glu Gly Glu
    450                 455                 460

Gly Asn Tyr Glu Leu Val Gly Ile Arg Asp Gln Gln Arg Gln Gln Asp
465                 470                 475                 480

Glu Gln Glu Glu Glu Glu Glu Glu Val Arg Arg Tyr Ser Ala Arg Leu
                485                 490                 495

Ser Glu Gly Asp Ile Phe Val Ile Pro Ala Gly Tyr Pro Ile Ser Val
            500                 505                 510

Asn Ala Ser Ser Asn Leu Arg Leu Leu Gly Phe Gly Ile Asn Ala Asn
        515                 520                 525

Glu Asn Gln Arg Asn Phe Leu Ala Gly Ser Glu Asp Asn Val Ile Ser
    530                 535                 540

Gln Leu Asp Arg Glu Val Lys Glu Leu Thr Phe Pro Gly Ser Ala Gln
545                 550                 555                 560

Asp Val Glu Arg Leu Ile Lys Asn Gln Gln Gln Ser Tyr Phe Ala Asn
                565                 570                 575

Ala Gln Pro Gln Gln Lys Gln Gln Arg Glu Lys Glu Gly Arg Arg Gly
            580                 585                 590

Arg Arg Ser Leu Ile Ser Ser Ile Leu Ser Thr Leu Tyr
        595                 600                 605

<210>  9
<211>  533
<212>  PRT
<213>  Lupinus albus

<400>  9
   Met Gly Lys Met Arg Val Arg Phe Pro Thr Leu Val Leu Val Leu Gly
   1               5                   10                  15
   Ile Val Phe Leu Met Ala Val Ser Ile Gly Ile Ala Tyr Gly Glu Lys
               20                  25                  30
   Asp Val Leu Lys Ser His Glu Arg Pro Glu Glu Arg Glu Gln Glu Glu
           35                  40                  45
   Trp Gln Pro Arg Arg Gln Arg Pro Gln Ser Arg Arg Glu Glu Arg Glu
       50                  55                  60
   Gln Glu Gln Glu Gln Gly Ser Pro Ser Tyr Pro Arg Arg Gln Ser Gly
   65                  70                  75                  80
   Tyr Glu Arg Arg Gln Tyr His Glu Arg Ser Glu Gln Arg Glu Glu Arg
                   85                  90                  95
   Glu Gln Glu Gln Gln Gln Gly Ser Pro Ser Tyr Ser Arg Arg Gln Arg
               100                 105                 110
   Asn Pro Tyr His Phe Ser Ser Gln Arg Phe Gln Thr Leu Tyr Lys Asn
           115                 120                 125
   Arg Asn Gly Lys Ile Arg Val Leu Glu Arg Phe Asp Gln Arg Thr Asn
       130                 135                 140
   Arg Leu Glu Asn Leu Gln Asn Tyr Arg Ile Val Glu Phe Gln Ser Lys
   145                 150                 155                 160
   Pro Asn Thr Leu Ile Leu Pro Lys His Ser Asp Ala Asp Tyr Val Leu
                   165                 170                 175
   Val Val Leu Asn Gly Arg Ala Thr Ile Thr Ile Val Asn Pro Asp Arg
               180                 185                 190
   Arg Gln Ala Tyr Asn Leu Glu Tyr Gly Asp Ala Leu Arg Ile Pro Ala
   195 200 205
   Gly Ser Thr Ser Tyr Ile Leu Asn Pro Asp Asp Asn Gln Lys Leu Arg
   210 215 220
   Val Val Lys Leu Ala Ile Pro Ile Asn Asn Pro Gly Tyr Phe Tyr Asp
   225 230 235 240
   Phe Tyr Pro Ser Ser Thr Lys Asp Gln Gln Ser Tyr Phe Ser Gly Phe
   245 250 255
   Ser Arg Asn Thr Leu Glu Ala Thr Phe Asn Thr Arg Tyr Glu Glu Ile
   260 265 270
   Gln Arg Ile Ile Leu Gly Asn Glu Asp Glu Gln Glu Tyr Glu Glu Gln
   275 280 285
   Arg Arg Gly Gln Glu Gln Ser Asp Gln Asp Glu Gly Val Ile Val Ile
   290 295 300
   Val Ser Lys Lys Gln Ile Gln Lys Leu Thr Lys His Ala Gln Ser Ser
   305 310 315 320
   Ser Gly Lys Asp Lys Pro Ser Asp Ser Gly Pro Phe Asn Leu Arg Ser
   325 330 335
   Asn Glu Pro Ile Tyr Ser Asn Lys Tyr Gly Asn Phe Tyr Glu Ile Thr
   340 345 350
   Pro Asp Arg Asn Pro Gln Val Gln Asp Leu Asn Ile Ser Leu Thr Tyr
   355 360 365
   Ile Lys Ile Asn Glu Gly Ala Leu Leu Leu Pro His Tyr Asn Ser Lys
   370 375 380
   Ala Ile Tyr Val Val Val Val Asp Glu Gly Glu Gly Asn Tyr Glu Leu
   385 390 395 400
   Val Gly Ile Arg Asp Gln Gln Arg Gln Gln Asp Glu Gln Glu Glu Lys
   405 410 415
   Glu Glu Glu Val Ile Arg Tyr Ser Ala Arg Leu Ser Glu Gly Asp Ile
   420 425 430
   Phe Val Ile Pro Ala Gly Tyr Pro Ile Ser Ile Asn Ala Ser Ser Asn
   435 440 445
   Leu Arg Leu Leu Gly Phe Gly Ile Asn Ala Asp Glu Asn Gln Arg Asn
   450 455 460
   Phe Leu Ala Gly Ser Lys Asp Asn Val Ile Arg Gln Leu Asp Arg Ala
   465 470 475 480
   Val Asn Glu Leu Thr Phe Pro Gly Ser Ala Glu Asp Ile Glu Arg Leu
   485 490 495
   Ile Lys Asn Gln Gln Gln Ser Tyr Phe Ala Asn Gly Gln Pro Gln Gln
   500 505 510
   Gln Gln Gln Gln Gln Ser Glu Lys Glu Gly Arg Arg Gly Arg Arg Gly
   515 520 525
   Ser Ser Leu Pro Phe
   530